# EUROPEAN PATENT APPLICATION

(11) **EP 4 578 943 A1**
(43) Date of publication of application: **02.07.2025**
(21) Application number: 23857430.5
(22) Date of filing: 25.08.2023
(51) Int. Cl.: C12N 5/10, C12N 7/01, C12N 15/864

(54) **METHOD FOR PRODUCING TARGE VIRUSES AND CULTURE COMPOSITION**

(30) Priority: 25.08.2022 JP 2022134297
(71) Applicant: FUJIFILM Corporation, Tokyo 106-8620 (JP)
(72) Inventor: MORI, Yusuke, Ashigarakami-gun, Kanagawa 258-8577 (JP); UMETANI, Sayako, Ashigarakami-gun, Kanagawa 258-8577 (JP); ONODERA, Keiichi, Ashigarakami-gun, Kanagawa 258-8577 (JP); MURAGUCHI, Taichi, Ashigarakami-gun, Kanagawa 258-8577 (JP)
(74) Representative: Hoffmann Eitle
(86) International application number: PCT/JP2023/030630
(87) International publication number: WO 2024/043322

(57) **Abstract**

An object of the present invention is to provide a method for producing a target virus, which is capable of improving a production amount of a virus, and a culture composition which contains an animal cell, a virus, and a culture medium and in which the production amount of the virus is improved. According to the present invention, there is provided a method of producing a target virus, including a virus production step of culturing an animal cell into which an exogenous recombinant nucleic acid encoding a target virus has been introduced, in a culture medium in a state in which a function of BET is suppressed.

## Description

### Technical Field

The present invention relates to a method of producing a target virus, the method including culturing an animal cell in a predetermined state. The present invention further relates to a culture composition containing an animal cell, a virus, and a culture medium.

### Background Art

The adeno-associated virus (AAV) is a linear single-stranded DNA virus belonging to Parvoviridae. The wild-type AAV genome contains a replication regulatory gene (Rep gene) and a capsid structural gene (Cap gene), and inverted terminal repeat sequences (ITR) are adjacent to these genes for viral replication and packaging. The AAV vector can introduce a gene to proliferating and non-proliferating cells, and can be expressed for a long term particularly in non-dividing cells. In addition, AAV is considered to be non-pathogenic and has low immunogenicity. From the above, the AAV vector has been clinically applied as a vector for gene therapy.

On the other hand, as a method of improving the production amount of the recombinant protein, Patent Document 1 describes that the production amount (titer) of an antibody is improved by treating Chinese hamster ovary cells (CHO cells) into which an antibody gene has been introduced with a bromodomain and extraterminal motif (BET) inhibitor. Patent Document 2 describes that the production of a virus of HSV infected in a human is improved by a bromodomain inhibitor. Patent Document 3 and Patent Document 4 describe a therapeutic method of reactivating latent HIV in cells to remove infected cells, and describe the use of an inhibitor of a bromodomain protein PCAF or JQ1.

### Prior Art Documents

### Patent Documents

Patent Document 1: WO2017/192691A
Patent Document 2: CN106551939A
Patent Document 3: US2015/0133434A
Patent Document 4: US2018/0002699A

### SUMMARY OF THE INVENTION

### Object to be solved by the invention

Currently, a gene therapy agent using AAV vectors have been put on the market, and there is a demand for the development of a production method that improves the production amount of viruses such as AAV vectors. An object to be achieved of the present invention is to provide a method of producing a target virus, which is capable of improving the production amount of the virus. Another object to be achieved of the present invention is to provide a culture composition containing an animal cell, a virus, and a culture medium, in which the production amount of the virus is improved.

### Means for solving the object

As a result of intensive studies to achieve the above object, the present inventors have found that the production amount of a target virus can be improved by culturing animal cells into which an exogenous recombinant nucleic acid encoding the target virus has been introduced, in a culture medium in a state in which the function of BET is suppressed. The present invention has been completed based on the above findings.

According to the present invention, the following inventions are provided.
<1> A method of producing a target virus, comprising a virus production step of culturing an animal cell into which an exogenous recombinant nucleic acid encoding a target virus has been introduced, in a culture medium in a state in which a function of BET is suppressed.
<2> The method according to <1>, in which transcription by an RNA polymerase III is promoted by suppressing the function of the BET.
<3> The method according to <1> or <2>, in which the function of the BET is suppressed by containing a BET inhibitor in the culture medium.
<4> The method according to any one of <1> to <3>, in which a dissociation constant: Kd value of a BET inhibitor with respect to any one of BRD2, BRD3, or BRD4 is 150 nmol/L or less.
<5> The method according to any one of <1> to <4>, in which a BET inhibitor is at least one selected from the group consisting of (S)-(+)-tert-butyl 2-(4-(4-chlorophenyl)-2,3,9-trimethyl-6H-thieno[3,2-f][1,2,4]triazolo[4,3-a][1,4]diazepin-6-yl)acetate,
   2-methoxy-N-(3-methyl-2-oxo-1,2,3,4-tetrahydro-quinazolin-6-yl)-benzenesulfonamide,
   N-[6-(3-methanesulfonamide-4-methylphenyl)-3-methyl-[1,2,4]triazolo[4,3-b]pyridazin-8-yl]carbamate,
   (4S)-6-(4-chlorophenyl)-N-ethyl-8-methoxy-1-methyl-4H-[1,2,4]triazolo[4,3-a][1,4]benzodiazepine-4-acetamide,
   7,3,5-dimethyl-4-isoxazolyl-1,3-dihydro-8-methoxy-1-[1R-1-(2-pyridinyl)ethyl]-2H-imidazo[4,5-c]quinolin-2-one, and
   6H-thieno[3,2-f][1,2,4]triazolo[4,3-a][1,4]diazepine-6-acetamide.
<6> The method according to any one of <1> to <5>, in which a BET inhibitor is at least one selected from the group consisting of (S)-(+)-tert-butyl 2-(4-(4-chlorophenyl)-2,3,9-trimethyl-6H-thieno[3,2-f][1,2,4]triazolo[4,3-a][1,4]diazepin-6-yl)acetate,
   2-methoxy-N-(3-methyl-2-oxo-1,2,3,4-tetrahydro-quinazolin-6-yl)-benzenesulfonamide,
   N-[6-(3-methanesulfonamide-4-methylphenyl)-3-methyl-[1,2,4]triazolo[4,3-b]pyridazin-8-yl]carbamate, and
   (4S)-6-(4-chlorophenyl)-N-ethyl-8-methoxy-1-methyl-4H-[1,2,4]triazolo[4,3-a][1,4]benzodiazepine-4-acetamide.
<7> The method according to any one of <3> to <6>, in which a concentration of the BET inhibitor in the culture medium is 0.01 µmol/L to 100 µmol/L.
<8> The method according to any one of <1> to <7>, in which the exogenous recombinant nucleic acid is expressed in a constitutive expression system in the animal cell.
<9> The method according to any one of <1> to <8>, in which at least one of the exogenous recombinant nucleic acids contains a promoter controlled by an RNA polymerase III.
<10> The method according to any one of <1> to <9>, in which the target virus is an adeno-associated virus.
<11> The method according to any one of <1> to <10>, in which the exogenous recombinant nucleic acid is a nucleic acid encoding E2A, E4, VA-RNA, Rep, and Cap.
<12> The method according to <9>, in which a gene that is transcribed from the promoter controlled by the RNA polymerase III is a VA-RNAI.
<13> The method according to any one of <1> to <12>, in which expression of at least one RNA which is a gene transcriptional product of the exogenous recombinant nucleic acid is 10 times or more higher as compared with a case of performing culturing in a state in which the function of the BET is not suppressed.
<14> The method according to any one of <1> to <13>, in which the exogenous recombinant nucleic acid includes at least a nucleic acid encoding E2A and VA-RNAI, and an RNA expression ratio between E2A gene and VA-RNAI gene is E2A:VA-RNAI = 1:1 to 1:10.
<15> The method according to any one of <1> to <14>, in which the animal cell is an HEK293 cell.
<16> The method according to any one of <1> to <15>, in which at least one of the exogenous recombinant nucleic acids contains a nucleic acid sequence that induces expression in a condition-specific manner.
<17> The method according to <16>, in which the expression is induced in a condition-specific manner by applying a stimulus that induces the expression in a condition-specific manner.
<18> The method according to any one of <1>to <17>, in which a stimulus that induces the expression in a condition-specific manner is that the culture medium contains doxycycline.
<19> The method according to any one of <1> to <18>, in which a period during which the culture medium contains a BET inhibitor is 1 hour or more.
<20> The method according to any one of <1> to <19>, in which the method includes stopping the suppression of the function of the BET before recovering a produced virus.
<21> The method according to <20>, in which the suppression of the function of the BET is stopped by performing culturing in a culture medium from which a BET inhibitor has been reduced or removed.
<22> The method according to <21>, in which the culturing is performed in the culture medium from which the BET inhibitor has been reduced or removed for 1 hour or more.
<23> The method according to any one of <1> to <22>, in which a culture medium is supplied during the culturing, and the culture medium to be supplied contains doxycycline and a BET inhibitor.
<24> The method according to any one of <1> to <23>, in which a cell density at the time of seeding the cells is 1 × 10⁴ cells/mL or more.
<25> The method according to any one of <1> to <24>, further comprising a proliferation step of proliferating the cells before the virus production step.
<26> The method according to <25>, in which a proliferation step of culturing the cells in a culture medium that does not contain doxycycline and a BET inhibitor until a cell density reaches 1 × 10⁴ cells/mL or more is provided before the virus production step, and the culture medium in the virus production step contains doxycycline and a BET inhibitor.
<27> The method according to any one of <1> to <26>, in which at least a part of the virus production step is perfusion culture.
<28> The method according to any one of <1> to <27>, in which a period of the virus production step is 24 hours or more and 30 days or less.
<29> The method according to any one of <1> to <28>, in which the method includes recovering the virus.
<30> A culture composition comprising an animal cell into which an exogenous recombinant nucleic acid encoding a target virus has been introduced and in which a function of BET is suppressed, a virus, and culture medium.
<31> The culture composition according to <30>, in which the virus is an adeno-associated virus.
<32> The culture composition according to <30> or <31>, in which the culture medium contains a BET inhibitor.

### Effect of the invention

According to the present invention, it is possible to improve the production amount of the target virus.

### BRIEF DESCRIPTION OF THE DRAWINGS

Fig. 1 shows an AAV production amount during compound treatment using cells cultured in a 24-well plate.
Fig. 2 shows a gene expression level during the compound treatment using cells cultured in a 24-well plate. Each column shows the expression of E4, VA-RNA, E2A, Rep, and Cap from the left.
Fig. 3 shows an AAV production amount during compound treatment using cells cultured in a 96-well plate. Each column shows the results of 1.35 µmol/L (1.35 µM in the figure), 0.45 µmol/L (0.45 µM in the figure), and 0.15 µmol/L (0.15 µM in the figure) from the left.
Fig. 4 shows the AAV production amount in the compound treatment (4 µmol/L (4 µM in the figure) of JQ1).

### EMBODIMENTS FOR CARRYING OUT THE INVENTION

Hereinafter, an example of the embodiment of the present disclosure will be described. However, the present disclosure is not limited to the following embodiments, and can be implemented with appropriate changes within the scope of the object of the present disclosure. In the present specification, a numerical range indicated using "to" means a range including numerical values described before and after "to" as a minimum value and a maximum value, respectively.

### <Explanation of terms>

A promoter means a DNA regulatory region/sequence to which an RNA polymerase can be bound and which is involved in the initiation of transcription of a downstream coding sequence or a downstream non-coding sequence.

The vector is a DNA or RNA molecule that is used to artificially transport an exogenous gene to another cell. A vector for expressing a gene is referred to as an expression vector. In a case where a vector containing an exogenous gene to be introduced is introduced into a cell, the exogenous gene is replicated and/or expressed in the cell. Examples of the vector include an episomal (for example, plasmid) vector and a non-episomal vector. The vector can be introduced into a host cell by methods such as transfection, transduction, cell fusion, and lipofection.

In the present specification, a gene means a nucleic acid molecule encoding a polypeptide or RNA, and examples thereof include a nucleic acid molecule such as cDNA or genomic DNA.

### <Method for producing target virus>

The method for producing a target virus according to the embodiment of the present invention includes a virus production step of culturing an animal cell into which an exogenous recombinant nucleic acid encoding a target virus has been introduced, in a culture medium in a state in which a function of BET is suppressed.

Examples of the method of suppressing the function of BET include (1) suppression of the function of BET by a treatment with a BET inhibitor (preferably a bromodomain inhibitor), (2) inhibition of expression of BET by siRNA or gene editing (addition of a mutation to a BET-related gene or change in an expression level), and (3) competitive inhibition by overexpression of a peptide or a protein. In addition, examples thereof include a method of suppressing the function of BET by controlling a protein (for example, acetylated histone or P-TEFb) upstream or downstream of the molecular signal by BET using the same method as described above. Among the above, suppression of the function of BET by treatment with a BET inhibitor (preferably a bromodomain inhibitor) is preferable.

The bromodomain is a protein domain that has a function known to recognize acetylated lysine of histone and to control a chromatin structure and gene expression by collecting control proteins. In humans, about 50 types of bromodomain-containing proteins exist. Examples of the protein containing a bromodomain include a bromodomain and extraterminal family protein (BET) having a bromodomain repeat sequence and a specific terminal sequence. As the BET, bromodomain-containing protein 2 (BRD2), BRD3, BRD4, and BRDT are known. These proteins each have a bromodomain 1 (BD 1) and a bromodomain 2 (BD2), which are N-terminal bromodomains.

Preferably, the function of BET can be suppressed by containing a BET inhibitor (preferably, a bromodomain inhibitor) in the culture medium. The BET inhibitor (preferably a bromodomain inhibitor) is a substance that binds to a bromodomain of a protein and inhibits the function of the bromodomain. BRD2, BRD3, and BRD4 have a bromodomain, and the function of BRD2, BRD3, and/or BRD4 is inhibited by a BET inhibitor (preferably a bromodomain inhibitor).

For the BET inhibitor (preferably a bromodomain inhibitor), the equilibrium dissociation constant: Kd value with respect to any one of BRD2, BRD3, or BRD4 is preferably 150 nmol/L or less, more preferably 100 nmol/L or less, and still more preferably 50 nmol/L or less.

Preferably, the Kd value with respect to the bromodomain 1 within BRD4 is 100 nmol/L or less, and the Kd value with respect to the bromodomain 2 is 250 nmol/L or less. More preferably, the Kd value with respect to the bromodomain 1 is 70 nmol/L or less, and the Kd value with respect to the bromodomain 2 is 200 nmol/L or less.

In a determination device of surface plasmon resonance (SPR), in a case where polarized light is irradiated to a surface of a metal thin film of a sensor chip such that total reflection occurs, an SPR signal in which the intensity of reflected light decreases is observed in a part of the reflected light. Since the angle at which the SPR signal occurs changes depending on the mass on the sensor chip, the angle at which the SPR signal occurs changes as the ligand immobilized on the sensor chip and the added analyte are bound or dissociated. The measurement result can be acquired as a sensor gram showing a change in angle of the SPR signal or a response unit (RU) converted from the change in angle over time. By fitting the acquired sensor grams of binding and dissociation to the theoretical curve, the binding rate constant (ka) and the dissociation rate constant (kd) between the ligand and the analyte can be determined, and further, the equilibrium dissociation constant (Kd = kd/ka) can be determined.

For the BET inhibitor (preferably a bromodomain inhibitor), the IC50 with respect to any one of BRD2, BRD3, or BRD4 is preferably 1 µmol/L or less, more preferably 500 nmol/L or less, still more preferably 200 nmol/L or less, even still more preferably 100 nmol/L or less, and particularly preferably 50 nmol/L or less.

The IC50 with respect to any of BRD2, BRD3, or BRD4 can be determined by plotting the binding inhibition value between the acetylated histone and the bromodomain against the concentration of the inhibitor and calculating the concentration corresponding to an inhibition rate of 50%.

Examples of the BET inhibitor (preferably, a bromodomain inhibitor) include (+)-JQ1 (also known as (S)-(+)-tert-butyl 2-(4-(4-chlorophenyl)-2,3,9-trimethyl-6H-thieno[3,2-f][1,2,4]triazolo[4,3-a][1,4]diazepin-6-yl)acetate [Cas#: 1268524-70-4]), PFI-1 (also known as 2-methoxy-N-(3-methyl-2-oxo-1,2,3,4-tetrahydro-quinazolin-6-yl)-benzenesulfonamide [Cas#: 1403764-72-6]), Bromosporine (also known as N-[6-(3-methanesulfonamido-4-methylphenyl)-3-methyl-[1,2,4]triazolo[4,3-b]pyridazin-8-yl]carbamate [Cas#: 1619994-69-2]), I-BET762 (also known as (4S)-6-(4-chlorophenyl)-N-ethyl-8-methoxy-1-methyl-4H-[1,2,4]triazolo[4,3-a][1,4]benzodiazepine-4-acetamide [Cas#: 1260907-17-2]), I-BET151 (also known as 7,3,5-dimethyl-4-isoxazolyl-1,3-dihydro-8-methoxy-1-[1R-1-(2-pyridinyl)ethyl]-2H-imidazo[4,5-c]quinolin-2-one [Cas#: 1300031-49-5]), and OTX015 (also known as 6H-thieno[3,2-f][1,2,4]triazolo[4,3-a][1,4]diazepine-6-acetamide [Cas#: 202590-98-5]). Examples of the other compounds include CPI-203 [Cas#: 1446144-04-2], RVX-208 [Cas#: 1044870-39-4], BI-2536 [Cas#: 755038-02-9], and AZD5153 [Cas#: 1869912-39-9].

Among the above, (+)-JQ1, PFI-1, Bromosporine, I-BET762, I-BET151, and OTX015 are preferable, (+)-JQ1, PFI-1, Bromosporine, and I-BET762 are more preferable, and (+)-JQ1 and PFI-1 are still more preferable.

The concentration of the BET inhibitor (preferably, bromodomain inhibitor) in the culture medium is preferably 0.01 µmol/L to 100 µmol/L, more preferably 0.05 µmol/L to 100 µmol/L, still more preferably 0.1 µmol/L to 50 µmol/L, and particularly preferably 1 µmol/L to 10 µmol/L.

The treatment period with the BET inhibitor (preferably, the bromodomain inhibitor), that is, the period in which the BET inhibitor (preferably, the bromodomain inhibitor) is contained in the culture medium is preferably 1 hour or more and 240 hours or less, more preferably 6 hours or more and 240 hours or less, still more preferably 12 hours or more and 240 hours or less, even still more preferably 24 hours or more and 120 hours or less, and particularly preferably 48 hours or more and 96 hours or less. The upper limit of the period in which a BET inhibitor (preferably a bromodomain inhibitor) is contained in the culture medium is not particularly limited, but is generally 240 hours or less, preferably 120 hours or less, and particularly preferably 96 hours or less.

In a case where a doxycycline-inducing promoter is used for expressing the exogenous recombinant nucleic acid, the culture medium may contain doxycycline. The concentration of doxycycline is preferably 5 µg/mL or less, more preferably 1 µg/mL or less, still more preferably 700 ng/mL or less, and particularly preferably 500 ng/mL or less.

The addition time of doxycycline is preferably 1 hour or more, more preferably 6 hours or more, still more preferably 12 hours or more, even still more preferably 24 hours or more, and yet even still more preferably 48 hours or more.

In the present invention, the culture medium is preferably supplied during the culturing, and the supplied culture medium may contain doxycycline and a BET inhibitor (preferably a bromodomain inhibitor).

The supplied culture medium is a culture medium to be added during cell culturing. The culture method using the supplied culture medium is not particularly limited, but perfusion culture is desirable.

The period for which doxycycline and the BET inhibitor (preferably, the bromodomain inhibitor) are allowed to act is preferably 1 hour or more, more preferably 6 hours or more, still more preferably 12 hours or more, even still more preferably 24 hours or more, and yet even still more preferably 48 hours or more.

The method according to the embodiment of the present invention may include stopping the suppression of the function of BET before the produced virus is recovered. The fact that the suppression of the function of BET is stopped means the removal of a factor that inhibits the expression of BET or the removal of an inhibitor from the culture medium. The method of the removal is not particularly limited, but it is preferable to completely or partially replace the culture medium in which the cells are being cultured. Preferably, the function of BET can be stopped by performing culturing in a culture medium in which the BET inhibitor (preferably, a bromodomain inhibitor) is reduced or removed. In this manner, it is possible to avoid the mixing of the BET inhibitor (preferably, a bromodomain inhibitor) in the virus composition to be used as a gene therapy agent.

The period in which the suppression of the function of BET is stopped is preferably 24 to 72 hours.

The period of performing culturing in the culture medium in which the BET inhibitor (preferably, a bromodomain inhibitor) is reduced or removed by completely or partially replacing the culture medium in which the cells are being cultured is preferably 1 hour or more, more preferably 1 to 240 hours, still more preferably 12 to 120 hours, and particularly preferably 24 to 72 hours.

In addition, in a case where a stimulus that induces expression in a condition-specific manner as described later is applied while the function of BET is stopped, it is preferable that the stimulus that induces expression in a condition-specific manner is continued even after the suppression of the function of BET is stopped.

On the contrary, the stimulus that induces the expression in a condition-specific manner may be stopped with the stop of the function of the BET. In a case where the stimulus that induces the expression in a condition-specific manner is the addition of a tetracycline-based drug, the stimulus that induces the expression in a condition-specific manner can be stopped by replacing with a culture medium that does not contain the tetracycline-based drug to reduce the tetracycline-based drug from the culture medium. In addition, in a case where the tetracycline-based drug is doxycycline, the culture medium can be similarly replaced with a culture medium that does not contain doxycycline.

Preferably, the culturing is performed in a culture medium containing a BET inhibitor (preferably, a bromodomain inhibitor) and a tetracycline-based drug for 12 to 48 hours, then the culture medium is replaced with a culture medium not containing a BET inhibitor (preferably, a bromodomain inhibitor), and then the culturing is performed for 24 to 72 hours.

In the present invention, the transcription by an RNA polymerase III is preferably promoted by suppressing the function of BET. The RNA polymerase III is an RNA transcription factor present in eukaryotic cells. Examples of the promoter that promotes transcription by the RNA polymerase III include an H1 promoter and a U6 promoter. The promotion is preferably an increase in the RNA level by 2 times or more.

It is known that the VA-RNA described later is induced to be expressed by the RNA polymerase III.

In the present invention, an animal cell into which an exogenous recombinant nucleic acid encoding a target virus has been introduced is used.

The target virus means a virus that is produced by introducing an exogenous nucleic acid into a cell. Examples of the target virus include an adeno-associated virus, a lentivirus, a baculovirus, and a retrovirus, and among these, an adeno-associated virus is preferable.

Adeno-associated virus (AAV) refers to a small, incompletely replicative, non-enveloped virus containing single-stranded DNA having about 4,700 bases, from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue directivity. It is said that AAV1 has high gene introduction efficiency into muscle, liver, airway, central nervous system, and the like, AAV5 has high gene introduction efficiency into central nervous system, liver, retina, and the like, and AAV6 has high gene introduction efficiency into heart, muscle, liver, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

AAV is a non-enveloped virus that proliferates in the presence of helper viruses such as adenovirus and herpesvirus. In the preparation of AAV to be used for gene therapy or nucleic acid introduction, classically, AAV replication has been performed by co-infecting host cells with an adenovirus. In addition, a gene responsible for the helper action of adenovirus has been clarified, and a plasmid incorporating this gene has also been used. For example, a plasmid containing a Rep gene and a Cap gene, an adenovirus helper plasmid, and a plasmid containing a gene for treatment or prevention are simultaneously transfected into cells, and can thus be packaged as recombinant AAV (rAAV).

The Rep gene and the Cap gene encode proteins involved in the replication and packaging of the virion. In the wild type, the Rep gene is expressed from the P5 promoter and the p19 promoter. The Cap region expresses VP1, VP2, and VP3. Examples of the promoter that is naturally carried by the Cap gene can include a p40 promoter.

The adeno-associated virus gene (such as a Rep gene and a Cap gene) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base are made to the wild-type adeno-associated virus gene (such as a Rep gene and a Cap gene), the number of the modified bases is preferably 1 to 20, more preferably 1 to 10, and still more preferably 1 to 3. The modified base sequence of the adeno-associated virus gene exhibits preferably 85% or more sequence identity, more preferably 90% or more sequence identity, still more preferably 95% or more sequence identity, and even still more preferably 98% or more sequence identity with the base sequence of the wild-type adeno-associated virus gene.

In a case where the Rep gene and the Cap gene are introduced into a cell, a vector containing the Rep gene and the Cap gene can be introduced into the cell. The arrangement of the Rep gene and the Cap gene in a vector is not particularly limited, the Rep gene may be located upstream of the Cap gene or downstream of the Cap gene, and the Rep gene is located preferably upstream of the Cap gene.

The Rep gene means a region of the AAV genome encoding a virus replication protein that is known to those skilled in the art and that is collectively required for replication of a virus genome, or its functional homolog, for example, a human herpesvirus 6 (HHV-6) Rep gene (mediating AAV-2 DNA replication is known). Accordingly, the coding region of the Rep gene includes at least a gene encoding REP78 and REP68 (long form of REP protein) and REP52 and REP40 (short form of REP protein) of AAV, or a functional homologue thereof. The coding region of the Rep gene used in the present invention may be derived from any AAV serum type, but is preferably derived from AAV2. Examples of those derived from AAV2 include REP78 and REP68, and REP52 and REP40, and ITR.

The Cap gene means a region in the AAV genome encoding a capsid protein of a virus known to those skilled in the art. Examples of these capsid proteins are AAV capsid proteins VP1, VP2 and VP3. The Cap gene used in the present invention may be derived from any AAV serum type, but is preferably derived from AAV2 or AAV5. AAV5 is particularly preferable.

As the vector containing the Rep gene and the Cap gene, for example, a plasmid, a nucleic acid sequence derived from a virus, or an artificially designed nucleic acid can be used, and a plasmid is preferable.

In the present invention, a gene for treatment or prevention may be introduced into cells. It is preferable that the gene for treatment or prevention is introduced in a state of being sandwiched between ITRs.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as AIDS, cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The gene for therapy or prophylaxis may be linked to a promoter for expressing the gene for therapy or prophylaxis. The promoter for expressing a gene for treatment or prevention is not particularly limited, but examples thereof can include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, and a phosphoglycerate kinase (PGK) promoter. The gene for treatment or prevention and the promoter for expressing the gene are preferably flanked by the ITR sequences.

In the present invention, it is preferable that a virus helper gene derived from an adenovirus is introduced into cells. The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

Examples of the virus helper gene derived from adenovirus can include E1A, E1B, E2A, E4, and VA-RNA. In the host cell having all or a part of the E1 region, the region of the adenovirus genome required for replicating the AAV genome and packaging of the AAV genome into the capsid to form a virus virion is the E2A region, the E4 region, and the VA-RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene, or a VA-RNA gene. The VA-RNA gene is preferably a VA-RNAI gene.

The adenovirus-derived virus helper gene (such as an E1A, an E1B, an E2A, an E4, and a VA-RNA) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base is made to the wild-type virus helper gene, the number of the modified bases is preferably 1 to 20, more preferably 1 to 10, and still more preferably 1 to 3. The modified base sequence of the virus helper gene exhibits preferably 85% or more sequence identity, more preferably 90% or more sequence identity, still more preferably 95% or more sequence identity, and even still more preferably 98% or more sequence identity with the base sequence of the wild-type virus helper gene.

**In** a case of introducing a virus helper gene into a cell, a vector containing the virus helper gene can be introduced into the cell.

As the vector containing a virus helper gene, for example, a plasmid, a virus-derived sequence, an artificially designed nucleic acid, or the like can be used, and a plasmid is preferable.

The virus helper gene is preferably under the control of a promoter and may be under the control of a promoter capable of regulating expression.

As the promoter capable of regulating expression, a promoter capable of regulating expression on and off by the presence or absence of a stimulus that induces expression in a condition-specific manner can be used. Examples of the stimulus that induces expression in a condition-specific manner include a chemical stimulus and a physical stimulus, but the present invention is not particularly limited thereto. Examples of the chemical stimulus include endogenous hormones, stress responses, lactose, tetracycline or a derivative thereof (for example, doxycycline), cumic acid, rapamycin, FKCsA, abscisic acid (ABA), tamoxifen/Cre-loxP (system in which a Cre promoter modified to be able to induce activation by tamoxifen is used), a riboswitch, and the like. Examples of the physical stimulus include blue light, heat, and the like.

Among the above, the promoter capable of regulating expression is preferably a promoter capable of regulating expression by a drug. The drug is preferably a tetracycline-based drug and more preferably doxycycline. That is, the stimulus that induces the expression in a condition-specific manner may be that the culture medium contains doxycycline.

Specifically, examples of the promoter capable of regulating expression include a tetracycline-responsive promoter, a RU486-inductive promoter, an ecdysone-inductive promoter, a rapamycin-inductive promoter, and a metallothionein promoter. Specific examples of the tetracycline-responsive promoter include a Tet-on/off system (manufactured by TET systems GmbH & Co. KG).

The promoter capable of regulating expression may be a Tet-on/off system which is a promoter capable of regulating expression by tetracycline, or a Tet-on system.

In the Tet-on system, the promoter additionally contains at least one Tet operon. By using a Tet operon (tetracycline-controlled transcriptional activation), transcription can be reversibly switched on or off in the presence of one of the tetracycline which is an antibiotic, or a derivative thereof (for example, doxycycline). The Tet repressor protein present in the cell blocks expression by binding to a Tet operator sequence introduced into a promoter. Therefore, no gene expression is observed in a case where the Tet repressor binds to the Tet operator sequence. By adding tetracycline or doxycycline, the Tet repressor is sequestered to allow promoter activity and turn on gene expression. The Tet operon system is widely available, such as the Tet operon used in the pcDNA (trademark) 4/TO mammalian expression vector available from Invitrogen.

In the Tet-on system, the Tet operator sequence may be located upstream or downstream of the promoter for which expression is desired to be regulated. The Tet operator sequence is preferably located downstream of a promoter capable of regulating expression in view of reducing expression leakage in a case where expression is turned off.

The specific examples of the promoter are not particularly limited, but can include a cytomegalovirus-derived promoter (CMV promoter) (including an enhancer as desired), an SV40 early promoter, a human elongation factor-1α (EF-1α) promoter, a human ubiquitin C promoter, a retrovirus Rous sarcoma virus (RSV) LTR promoter, a dihydrofolate reductase promoter, a β-actin promoter, and a phosphoglycerate kinase (PGK) promoter.

The exogenous recombinant nucleic acid means a recombinant nucleic acid introduced from the outside of a cell into the inside of the cell. The exogenous recombinant nucleic acid can be introduced into cells by methods such as transfection, transduction, and lipofection in a form of a vector containing the exogenous recombinant nucleic acid.

Transfection refers to a process of allowing a nucleic acid to cross a membrane of a eukaryotic cell and to be introduced into a cell by a chemical means (for example, using a reagent such as calcium phosphate or polyethyleneimine), a mechanical means (for example, electroporation), or a physical means (for example, delivery by bioballistic).

Transduction refers to introduce of a nucleic acid into a cell across a membrane of a eukaryotic cell via a virus-derived vector.

Lipofection refers to introduce of a nucleic acid into a cell by the formation of a complex between a vector and a positively charged lipid or the like by electrical interaction, and endocytosis or membrane fusion.

Preferably, in the animal cell, the exogenous recombinant nucleic acid is expressed in a constitutive expression system. The constitutive expression system is a method of expressing for 1 month or more. It is possible to maintain the expression of the target gene for a long period of time by inserting a plasmid into a chromosome, introducing an episomal vector, or introducing an exogenous nucleic acid using an artificial chromosome. In this case, in a case where the drug resistance gene is introduced into the plasmid, selection with a drug can be performed.

The recombinant nucleic acid is not particularly limited as long as it is a sequence capable of expressing a virus. Preferably, the recombinant nucleic acid is a sequence that expresses an adeno-associated virus. As described above, a sequence derived from a helper virus and a sequence derived from an adeno-associated virus is known as the sequence that expresses the adeno-associated virus, but the sequence is not limited to these as long as the adeno-associated virus can be expressed. The components derived from the helper virus include E1A, E1B, E2A, E4, and/or VA-RNA, but are not limited thereto. As the sequence derived from the adeno-associated virus, Rep and Cap are known, but the present invention is not limited thereto.

In the present invention, it is preferable that at least one of the exogenous recombinant nucleic acids contain a promoter controlled by an RNA polymerase III.

In the present invention, the exogenous recombinant nucleic acid is preferably a nucleic acid encoding E2A, E4, VA-RNA, Rep, and Cap.

In the present invention, the gene that is transcribed from a promoter controlled by the RNA polymerase III is more preferably VA-RNAi.

In the present invention, the expression of at least one RNA which is a gene transcriptional product of the exogenous recombinant nucleic acid is preferably 10 times or more, more preferably 10 times or more and 100 times or less, and still more preferably 20 times or more and 50 times or less, as compared with a case of culturing in a state in which the function of BET is not suppressed. Preferably, the expression of the VA-RNAI is 20 times or more. The state in which the function of BET is not suppressed is a state in which a BET inhibitor (preferably a bromodomain inhibitor) is not added, or a state in which an endogenous BET gene is not mutated or suppressed in expression, or a full length or a part of an exogenous wild-type or mutant BET gene is not introduced.

In the present invention, the exogenous recombinant nucleic acid preferably includes a nucleic acid encoding at least E2A and VA-RNAI, and the RNA expression ratio between the E2A and the VA-RNAI gene is preferably E2A:VA-RNAI = 1:1 to 1:20, more preferably E2A:VA-RNAI = 1:1 to 1:15, and still more preferably E2A:VA-RNAI = 1:1 to 1:10.

It is preferable that at least one of the exogenous recombinant nucleic acids contains a nucleic acid sequence that induces expression in a condition-specific manner. The promoter induced by a nucleic acid sequence that induces expression in a condition-specific manner is not particularly limited, but is preferably a doxycycline-inducible TET promoter.

As a manufacturing method of the adeno-associated virus, any of a triple transfection (TT) method, a packaging cell method, or a producer cell method may be used. From the viewpoint of the effects of the present invention, the packaging cell method or the Producer cell method is preferable, and the Producer cell method is most preferable.

The TT method is a method in which a plasmid containing a Rep gene and a Cap gene, an adenovirus helper plasmid, and a plasmid containing a transgene (for example, a gene for desired therapy or prophylaxis) are simultaneously transfected into cells, and can thus be packaged as recombinant AAV (rAAV).

The packaging cell method is a method of producing AAV by introducing, to the cell in which a part of the gene in the TT method is incorporated into a chromosome in advance, the remaining genes into a cell by transfection.

The Producer cell method is a method of producing AAV using cells that produce AAV without additional gene introduction. Preferably, the method is a method of producing AAV without a transfection operation using cells in which a gene (containing at least Rep and Cap genes) in the TT method is incorporated into a chromosome. More preferably, the Producer cell method is a method of producing AAV without a transfection operation using cells in which all genes in the TT method are incorporated into a chromosome.

The animal cell is not particularly limited, but is preferably a mammalian cell or an insect cell, and more preferably a mammalian cell. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, a human embryo-derived kidney (HEK293) cell, VERO, PER. C6, HeLa, EB1, EB2, EB3, and oncolytic or hybridoma cell lines. The cell is preferably a HEK293 cell or a CHO cell, and more preferably a HEK293 cell.

The method according to the embodiment of the present invention includes a virus production step of culturing animal cells in a culture medium.

The culturing the cells in the present invention can be performed under normal conditions for culturing the cells.

The cell density at the time of seeding the cells in the virus production step is preferably 1 × 10⁴ cells/mL or more, more preferably 1 × 10⁵ cells/mL or more, still more preferably 1 × 10⁶ cells/mL or more, even still more preferably 1 × 10⁷ cells/mL or more, and particularly preferably 1 × 10⁸ cells/mL or more. The upper limit of the cell density at the time of seeding the cells is not particularly limited, but is generally 1 × 10⁹ cells/mL or less.

In the present invention, a proliferation step of proliferating cells can be performed before the virus production step. The form of the culturing in the proliferation step is not particularly limited, but is preferably perfusion culture.

For example, a proliferation step of culturing cells in a culture medium that does not contain doxycycline and a BET inhibitor (preferably a bromodomain inhibitor) until the cell density reaches 1 × 10⁴ cells/mL or more (more preferably 1 × 10⁵ cells/mL or more, still more preferably 1 × 10⁶ cells/mL or more, even still more preferably 1 × 10⁷ cells/mL or more, and particularly preferably 1 × 10⁸ cells/mL or more) is performed before the virus production step, and then the virus production step can be performed using a culture medium containing doxycycline and a BET inhibitor (preferably a bromodomain inhibitor).

The culture temperature of the cells is not particularly limited, and the culture of the cells are performed at a temperature at which the cells can survive. The culture temperature is generally 25°C to 45°C, preferably 30°C to 42°C, more preferably 35°C to 40°C, and 37°C as an example.

The CO₂ concentration is generally 3 to 10% CO₂, preferably 5 to 10% CO₂, and 8% CO₂ as an example.

The period of the virus production step is preferably 24 hours or more and 30 days or less, more preferably 24 hours or more and 20 days or less, still more preferably 24 hours or more and 10 days or less, even still more preferably 24 hours or more and 7 days or less, and particularly preferably 24 hours or more and 72 hours or less.

As the culture, batch culture, fed-batch culture, perfusion culture, or shaking culture can be performed. It is preferable that at least a part of the virus production step is perfusion culture.

As the culture container, a flask or a bioreactor can be used, but it is not particularly limited.

The culture scale is not particularly limited, and the cells can be cultured in a culture medium of any volume, for example, the cells can be cultured in a culture medium of 1 mL to 2,000 L, preferably 1 L to 2,500 L, more preferably 50 L to 2,300 L, and particularly preferably 400 L to 2,200 L.

The culture may be carried out while stirring by shaking. The stirring speed in the case of stirring by shaking is generally 50 rpm to 200 rpm, and preferably 80 to 150 rpm. The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

Examples of the culture medium include, but are not limited to, Expi293 Expression Medium (Thermo Fisher Scientific, Inc., A1435101), a Dalbecco's modified Eagle's medium (DMEM) containing 10% (vol/vol) fetal bovine serum (FBS), a serum-free UltraCULTURE (trademark) culture medium (Lonza), a STEMPRO (trademark)-34 SFM culture medium, a human endothelial-SFM, a GIBCO (registered trademark) FREESTYLE (trademark) 293 expression medium, a CD CHO AGT culture medium, a CHO-S-SFM culture medium, a GIBCO (registered trademark) FREESTYLE (trademark) CHO expression medium, a CD OPTICHO (trademark) culture medium, a CD CHO culture medium, a CD DG44 culture medium, a SF-900 (trademark) culture medium, an Expi293 (trademark) expression medium, a 293 SFM culture medium, a BALANCD (registered trademark) HEK293 culture medium, and any derivates or modifications thereof. In a certain specific non-limiting embodiment, the high-density culture medium may be a CD FORTICHO (trademark) culture medium, a CD CHO AGT culture medium, a CHO-S-SFM culture medium, a GIBCO (registered trademark) FREESTYLE (trademark) CHO expression medium, a CD OPTICHO (trademark) culture medium, a CD CHO culture medium, a CD DG44 culture medium, a GIBCO (registered trademark) FREESTYLE (trademark) 293 expression medium, an Expi293 (trademark) expression medium, an LV-MAX (trademark) production culture medium, a FREESTYLE (trademark) F17 expression medium, a DYNAMIS (trademark) culture medium, a BALANCD (registered trademark) HEK293 culture medium, a similar culture medium, or a modified form thereof.

The culture medium is preferably Expi293 Expression Medium (Thermo Fisher Scientific, A1435101), or a Dulbecco's Modified Eagle's Medium (DMEM) containing 10% (vol/vol) fetal bovine serum (FBS).

The method for producing a target virus according to the embodiment of the present invention may include recovering the virus. The target virus produced in the cell that produces a virus remains in the cell or is released into the culture supernatant.

In a case of recovering a target virus from cells, for example, a specimen containing the target virus can be prepared by subjecting the cells to ultrasonic lysis, freezing and thawing the cells to lyse the cells, or bringing the cells into contact with an acidic solution. The specimen prepared in this way may be used as it is, or may be purified as desired.

In a case of recovering the target virus from the culture supernatant, the culture supernatant may be used as it is as a specimen containing the target virus, or may be purified as desired.

The purification method in a case of purifying the target virus is not particularly limited, and examples thereof include a CsCl gradient ultracentrifugation method, a chromatography method, and an ultrafiltration method. By the above-described purification method, the target virus can be purified from a specimen containing the target virus. Furthermore, the genome DNA (gDNA) or the residual plasmid may be digested by adding MgCl₂ and Benzonase to the specimen purified as described above to perform a reaction.

The titer of the target virus produced by the method according to the embodiment of the present invention can be measured by a normal method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding MgCl₂ and Benzonase to the collected supernatant to react. It is possible to measure the titer of a target virus by performing droplet digital PCR (ddPCR) using the sample containing the target virus obtained above as a ddPCR sample.

In an example of the present invention, by suppressing the function of BET, the production amount of a target virus such as AAV can be improved by 2 times or more as compared with the production method in the related art.

### <Culture composition>

According to the present invention, there is provided a culture composition containing an animal cell into which an exogenous recombinant nucleic acid encoding a target virus has been introduced and in which a function of BET is suppressed, a virus, and culture medium.

Specific examples and preferred aspects of the "animal cell in which an exogenous recombinant nucleic acid encoding a target virus has been introduced and a function of BET is suppressed", "virus", and "culture medium" are as described in the present specification.

In the culture composition according to the embodiment of the present invention, the virus is preferably an adeno-associated virus.

In the culture composition according to the embodiment of the present invention, the culture medium preferably contains a BET inhibitor (preferably a bromodomain inhibitor).

The present invention will be more specifically described using the following examples; however, it is not limited by the examples.

### Examples

### [Materials and methods]

### <Cell culture>

Floating HEK293 cells (Thermo Fisher Scientific, R79007) were suspended in 12 mL of Expi293 Expression Medium (Thermo Fisher Scientific, A1435101) in a 1 × 10⁷ cells/mL, and cultured in 125 mL shaking flask for 24 hours. The culture solution was incubated under the conditions of 37°C and 8% CO₂ while being constantly stirred at 120 rpm.

### <Production of plasmid-introduced cell>

HEK293 cells were seeded in a 24-well plate at a density of 0.75 × 10⁵ cells/well in a DMEM medium containing 10% FBS (clontech, 631101) (FUJIFILM Wako Pure Chemical Corporation, 041-29775), and then subjected to adhesion culture for 24 hours. The plasmids (seq1 and seq2) were co-introduced into HEK293 cells using TransIT-293 (Takara Bio, MIR2704) reagent. After 0.5 months, the cells were each seeded in a 96-well plate, and after further expansion culture for 1 month, an AAV producer cell strain in which the plasmid was incorporated into the chromosome was produced.

Seq1: all-in-one plasmid (integrated plasmid into which a gene required for AAV production is incorporated). The Rep gene and the Cap gene are controlled by a p5 promoter contained in the wild-type AAV. The E2 gene is controlled by a promoter contained in a wild-type adenovirus. The seq1 is designed as E4ORF6 is under the CMV promoter, and VA-RNA is under the H1 promoter, and the TET operator sequence is incorporated into the promoter to be capable of being controlled by expression by doxycycline.

Seq2: a plasmid (GENEWIZ) obtained by synthesizing the piggyBac transposase gene sequence and inserting the gene sequence into the EcoRV site of the pUC57 vector.

The base sequence of the seq1 is set forth in SEQ ID NO: 1 in the sequence list, and the base sequence of seq2 is set forth in SEQ ID NO: 2 in the sequence list.

### <Compound screening 1>

The produced AAV producer cell was seeded in a 24-well plate at 5 × 10⁵ cells/mL, incubated for 6 hours (the number of cells was 5 × 10⁵ cells/mL or more), and then doxycycline (final concentration of 0.5 µg/mL) and Epigenetics Screening Library (CAYMAN, 11076) (final concentration of 4 µmol/L) were added. After the treatment for 72 hours, the cells were recovered, and the evaluation of the Real-time PCR and the AAV titer measurement were performed.

### <Real-time PCR>

The HEK cells cultured 72 hours after the gene introduction under the conditions of 37°C and 8% CO₂ were collected by centrifugation at 300 x g. mRNA was purified using RNeasy Plus Mini Kit (Qiagen), and cDNA was prepared by reverse transcription using High Capacity RNA to cDNA Kit. The produced cDNA was quantitatively evaluated by detection with primers and probes for various genes (E2: seq3, 4, 5, VA-RNA: seq6, 7, 8, E4ORF6: seq9, 10, 11, Rep: seq12, 13, 14, and Cap: seq15, 16, 17) using TaqMan Gene Expression Master Mix (Applied Biosystems), and by standardization for GAPDH (Thermo Fisher Scientific, Hs02786624_g1) using TaqMan Gene Expression Assay. The base sequences of the seqs. 3 to 17 are each shown in SEQ ID NOs: 3 to 17 in the sequence list.

### <Measurement of AAV titer>

The HEK cell culture solution, which had been cultured for 72 hours after the gene introduction under the conditions of 37°C and 8% CO₂, was collected, and the cells were disrupted by freezing and thawing at -80°C. Then, the supernatant was collected by centrifugation at 13,800 ×g, 2 mmol/L (final concentration) MgCl₂, and 25 U/mL Benzonase was added thereto, and a reaction was carried out at 37°C for 2 hours to digest gDNA (genomic DNA) and residual plasmid. The sample after the reaction was incubated at 95°C for 15 minutes, 70°C for 3 minutes, 40°C for 3 minutes, and 20°C for 3 minutes in this order, to inactivate Benzonase, and the reactant was used as a ddPCR (Droplet Digital PCR) sample. The ddPCR sample was diluted 50-fold with a solution in which 0.05% Pluronic F-68 and 10 µg/mL bovine thymus DNA was mixed to a TE buffer (pH 8.0). 10 µL of ddPCRtm Supermix for Probes (no dUTP) (Bio-Rad Laboratories, Inc.), 2 µL of diluted ddPCR sample, primers (seq18, seq19, final concentration of 900 nmol/L) and probe (seq20, final concentration of 250 nmol/L), nuclease free water (Thermo Fisher Scientific, 10977015) was mixed in a tube on ice, the total liquid volume was adjusted to 22 µL, and ddPCR was performed. The sequences of the seq18, seq19, and seq20 are set forth in SEQ ID NOs: 18 to 20 in the sequence list.

In ddPCR, droplets were formed from the prepared solution using a droplet forming apparatus, and subsequently incubated at 95°C for 10 minutes, 94°C for 30 seconds, and 55°C in this order for 40 cycles, and then incubated at 98°C for 10 minutes. Measurement was performed using a droplet reader, and the AAV genomic titer (vg/mL) was calculated.

### <Compound screening 2>

The produced AAV producer cell was seeded in a 96-well plate at 5 × 10⁵ cells/mL, incubated for 6 hours (the number of cells was 5 × 10⁵ cells/mL or more), and then doxycycline (final concentration of 0.5 µg/mL) and Epigenetics Screening Library (CAYMAN, 11076) (final concentrations of 0.15, 0.45, and 1.35 µmol/L) were added. After the treatment for 72 hours, the cells were recovered, and the AAV titer was evaluated.

### [Result]

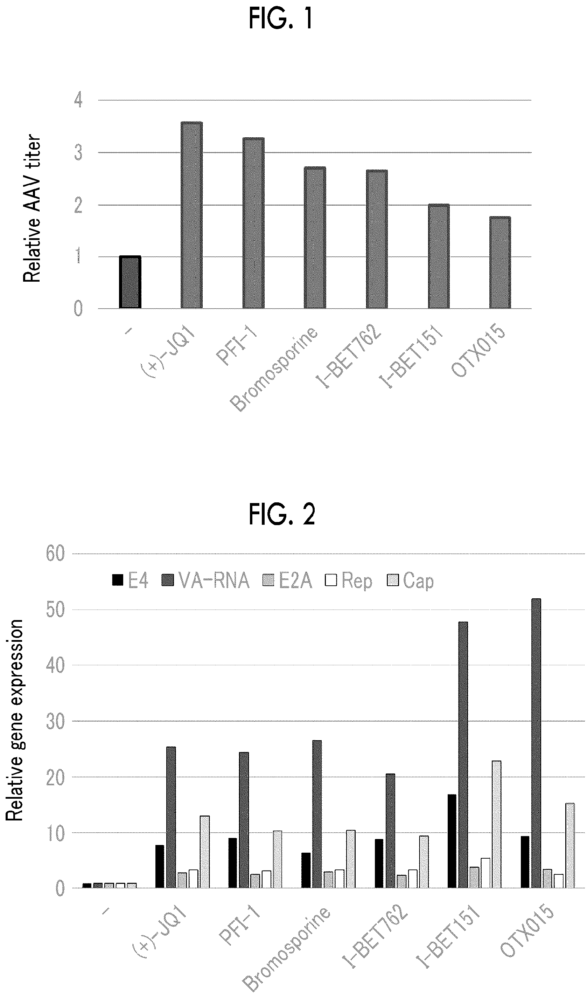

The change in the AAV production amount at the time of each bromodomain inhibitor addition was evaluated. Table 1 and Fig. 1 show the results of measuring the titer of AAV by ddPCR in Compound screening 1, and Table 2 and Fig. 2 show the gene expression by qPCR.

**[Table 1]**

| Relative AAV titer | |
|---|---|
| Treatment compound | AAV titer (relative value) |
| - | 1 |
| (+)-JQ1 | 3.6 |
| PFI-1 | 3.3 |
| Bromosporine | 2.7 |
| I-BET 762 | 2.6 |
| I-BET 151 | 2.0 |
| OTX 015 | 1.8 |

**[Table 2]**

| Gene expression level (relative value) | | | | | | | |
|---|---|---|---|---|---|---|---|
| | - | (+)-JQ1 | PFI-1 | Bromosporine | I-BET 762 | I-BET 151 | OTX 015 |
| E4 | 1 | 7.9 | 9.1 | 6.4 | 8.9 | 16.9 | 9.4 |
| VA-RNA | 1 | 25.4 | 24.5 | 26.5 | 20.7 | 47.8 | 51.9 |
| E2A | 1 | 2.9 | 2.6 | 3.0 | 2.5 | 4.0 | 3.4 |
| Rep | 1 | 3.4 | 3.2 | 3.3 | 3.4 | 5.5 | 2.6 |
| Cap | 1 | 13.0 | 10.4 | 10.5 | 9.5 | 22.9 | 15.3 |

From the results of Fig. 1 and Fig. 2, it was found that the gene expression level was accelerated and the AAV production amount was increased. The expression level of each gene was increased by 2.5 times or more under the condition without the addition of the inhibitor, and particularly, the expression of VA-RNA was increased by 20 times or more.

As the Kd value of the bromodomain inhibitor used, the nominal numerical value published by the reagent manufacturer is shown in Table 3.

As the IC50 value of the bromodomain inhibitor used, the nominal numerical value published by the reagent manufacturer is shown in Table 4.

**[Table 3]**

| Kd value of bromodomain inhibitor | | |
|---|---|---|
| | Kd of BRD4 (nmol/L) | |
| | Bromodomain 1 | Bromodomain 2 |
| (+)-JQ1 | 49.0 | 90.1 |
| PFI-1 | 47.4 | 195 |
| Bromosporine | 41.8 | 39.7 |
| I-BET 762 | 55.2 | |
| I-BET 151 | 100 | |
| OTX 015 | 10 | |

**[Table 4]**

| IC50 value of bromodomain inhibitor | |
|---|---|
| | IC50 of BRD4 (nM) |
| (+)-JQ1 | 17.7 |
| PFI-1 | 47.4 |
| Bromosporine | 122 |
| I-BET 762 | 36.1 |
| I-BET 151 | 790 |
| OTX 015 | 192 |

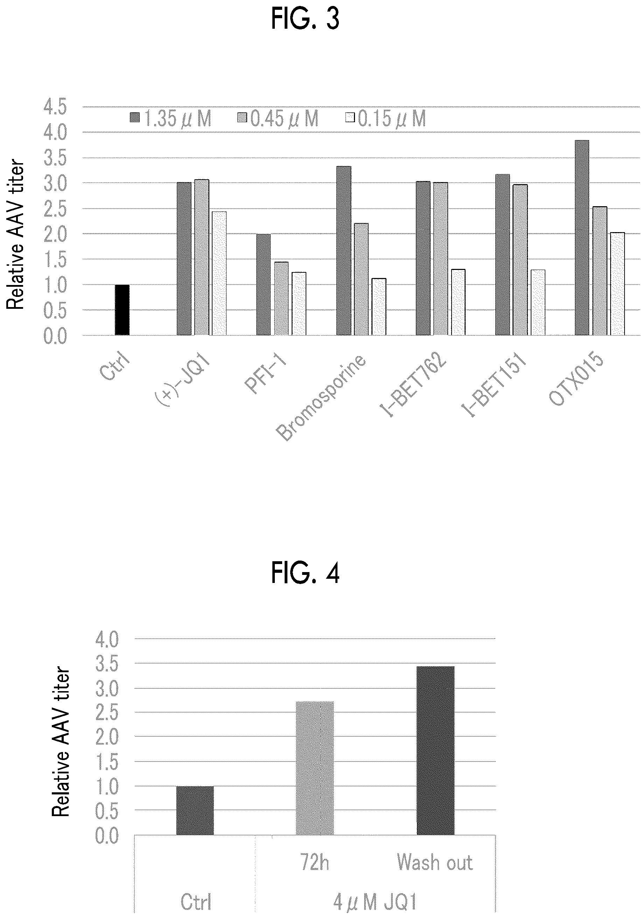

Fig. 3 shows the results of measuring the titer of AAV by ddPCR in Compound screening 2.

Furthermore, the produced AAV producer cell was seeded at 5 × 10⁵ cells/mL and incubated for 6 hours, and then doxycycline (final concentration: 0.5 µg/mL) and JQ1 (final concentration: 4 µmol/L) were added.

Fig. 4 shows the results of measuring the titer of AAV by ddPCR for the following samples.
Ctrl: a sample to which JQ1 was not added.
72h: a sample recovered after treatment with JQ1 for 72 hours.
Wash out: a sample subjected to the treatment with JQ1 for 24 hours and to a culture medium exchange of replacing with a culture medium containing no JQ1, and recovered further after 48 hours. (The culture medium after the exchange contained doxycycline (final concentration of 0.5 µg/mL))

After the JQ1 treatment, the AAV titer per cell was further improved by removing JQ1 from the culture medium.
Seq1: 
ATGACTCTCTTAAGGTAGCCAAATTTCCATAGGCTCCGCCCCCCTGACGAG
CATCACAAAAATCGACGCTCAAGTCAGAGGTGGCGAAACCCGACAGGACTATAAA
GATACCAGGCGTTTCCCCCTGGAAGCTCCCTCGTGCGCTCTCCTGTTCCGACCCTG
CCGCTTACCGGATACCTGTCCGCCTTTCTCCCTTCGGGAAGCGTGGCGCTTTCTCAT
AGCTCACGCTGTAGGTATCTCAGTTCGGTGTAGGTCGTTCGCTCCAAGCTGGGCTG
TGTGCACGAACCCCCCGTTCAGCCCGACCGCTGCGCCTTATCCGGTAACTATCGTCT
TGAGTCCAACCCGGTAAGACACGACTTATCGCCACTGGCAGCAGCCACTGGTAAC
AGGATTAGCAGAGCGAGGTATGTAGGCGGTGCTACAGAGTTCTTGAAGTGGTGGCC
TAACTACGGCTACACTAGAAGGACAGTATTTGGTATCTGCGCTCTGCTGAAGCCAG
TTACCTTCGGAAAAAGAGTTGGTAGCTCTTGATCCGGCAAACAAACCACCGCTGGT
AGCGGTGGTTTTTTTGTTTGCAAGCAGCAGATTACGCGCAGAAAAAAAGGATCTCA
AGAAGATCCTTTGATCTTTTCTACGGGGTCTGACGCTCAGTGGAACGAAAACTCAC
GTTAAGGGATTTTGGTCATGAGATTATCAAAAAGGATCTTCACCTAGATCCTTTTAA
ATTAAAAATGAAGTTTTAAATCAATCTAAAGTATATATGAGTAAACTTGGTCTGACA
GTTACCAATGCTTAATCAGTGAGGCACCTATCTCAGCGATCTGTCTATTTCGTTCATC
CATAGTTGCCTGACTCCCCGTCGTGTAGATAACTACGATACGGGAGGGCTTACCATC
TGGCCCCAGTGCTGCAATGATACCGCGAGACCCACGCTCACCGGCTCCAGATTTAT
 
CAGCAATAAACCAGCCAGCCGGAAGGGCCGAGCGCAGAAGTGGTCCTGCAACTTT
ATCCGCCTCCATCCAGTCTATTAATTGTTGCCGGGAAGCTAGAGTAAGTAGTTCGCC
AGTTAATAGTTTGCGCAACGTTGTTGCCATTGCTACAGGCATCGTGGTGTCACGCTC
GTCGTTTGGTATGGCTTCATTCAGCTCCGGTTCCCAACGATCAAGGCGAGTTACATG
ATCCCCCATGTTGTGCAAAAAAGCGGTTAGCTCCTTCGGTCCTCCGATCGTTGTCAG
AAGTAAGTTGGCCGCAGTGTTATCACTCATGGTTATGGCAGCACTGCATAATTCTCT
TACTGTCATGCCATCCGTAAGATGCTTTTCTGTGACTGGTGAGTACTCAACCAAGTC
ATTCTGAGAATAGTGTATGCGGCGACCGAGTTGCTCTTGCCCGGCGTCAATACGGG
ATAATACCGCGCCACATAGCAGAACTTTAAAAGTGCTCATCATTGGAAAACGTTCTT
CGGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTGA
GCAAAAACAGGAAGGCAAAATGCCGCAAAAAAGGGAATAAGGGCGACACGGAAA
TGTTGAATACTCATACTCTTCCTTTTTCAATATTATTGAAGCATTTATCAGGGTTATTG
TCTCATGAGCGGATACATATTTGAATGTATTTAGAAAAATAAACAAATAGGGGTTCC
GCGCACATTTCCCCGAAAAGTGCCACCTAAATTGTAAGCGTTAATATTTTGTTAAAA
TTCGCGTTAAATTTTTGTTAAATCAGCTCATTTTTTAACCAATAGGCCGAAATCGGC
AAAATCCCTTATAAATCAAAAGAATAGACCGAGATAGGGTTGAGTGTTGTTCCAGT
TTGGAACAAGAGTCCACTATTAAAGAACGTGGACTCCAACGTCAAAGGGCGAAAA
ACCGTCTATCAGGGCGATGGCCCACTACGTGAACCATCACCCTAATCAAGTTTTTTG
GGGTCGAGGTGCCGTAAAGCACTAAATCGGAACCCTAAAGGGAGCCCCCGATTTA
GAGCTTGACGGGGAAAGCCGGCGAACGTGGCGAGAAAGGAAGGGAAGAAAGCG
AAAGGAGCGGGCGCTAGGGCGCTGGCAAGTGTAGCGGTCACGCTGCGCGTAACCA
CCACACCCGCCGCGCTTAATGCGCCGCTACAGGGCGCGTCCCATTCGCCATTCAGG
CTGCGCAACTGTTGGGAAGGGCGATCGGTGCGGGCCTCTTCGCTATTACGCCAGCT
GGCGAAAGGGGGATGTGCTGCAAGGCGATTAAGTTGGGTAACGCCAGGGTTTTCC
CAGTCACGACGTTACCCCACAGTGGGGCCACTAGGGTCAATTTCTGAGAACTGTCA
TTCTCGGAAATTGAGTAAAACGACGGCCAGTGAGCGCGCCTCGTTCATTCACGTTT
TTGAACCCGTGGAGGACGGGCAGACTCGCGGTGCAAATGTGTTTTACAGCGTGAT
GGAGCAGATGAAGATGCTCGACACGCTGCAGAACACGCAGCTAGATTAACCCTAG
AAAGATAATCATATTGTGACGTACGTTAAAGATAATCATGCGTAAAATTGACGCATG
TGTTTTATCGGTCTGTATATCGAGGTTTATTTATTAATTTGAATAGATATTAAGTTTTAT
TATATTTACACTTACATACTAATAATAAATTCAACAAACAATTTATTTATGTTTATTTAT
TTATTAAAAAAAAACAAAAACTCAAAATTTCTTCTATAAAGTAACAAAACTTTTATC
 
GAATTCCTGCAGCCCGGGGGATCCACTAGTTCTAGAGGGACAGCCCCCCCCCAAA
GCCCCCAGGGATGTAATTACGTCCCTCCCCCGCTAGGGGGCAGCAGCGAGCCGCCC
GGGGCTCCGCTCCGGTCCGGCGCTCCCCCCGCATCCCCGAGCCGGCAGCGTGCGG
GGACAGCCCGGGCACGGGGAAGGTGGCACGGGATCGCTTTCCTCTGAACGCTTCT
CGCTGCTCTTTGAGCCTGCAGACACCTGGGGGGATACGGGGAAAAGGCCTCCAAG
GCCAGCTTCCCACAATAAGTTGGGTGAATTTTGGCTCATTCCTCCTTTCTATAGGATT
GAGGTCAGAGCTTTGTGATGGGAATTCTGTGGAATGTGTGTCAGTTAGGGTGTGGA
AAGTCCCGCGATCGCTAGCGTTTAAACTTAAGCTTGGTACCGAGCTCGGATCCACT
AGTCCAGTGTGGTGGAATTCCTGCTTCGCGATGTACGGGCCAGATATACGCGTTGC
CCGGGCGACCGCACCCTGTGACGAAAGCCGCCCGCAAGCTGCGCCCCTGAGTTAG
TCATCTGAACTTCGGCCTGGGCGTCTCTGGGAAGTACCACAGTGGTGGGAGCGGG
ACTTTCCTGGTACACCAGGGCAGCGGGCCAACTACGGGGATTAAGGTTATTACGAG
GTGTGGTGGTAATAGCCGCCTGTTCGAGGAGAATTCGGTTTCGGTGGGCGCGGATT
CCGTTGACCCGGGATATCATGTGGGGTCCCGCGCTCATGTAGTTTATTCGGGTTGAG
TAGTCTTGGGCAGCTCCAGCCGCAAGTCCCATTTGTGGCTGGTAACTCCACATGTA
GGGCGTGGGAATTTCCTTGCTCATAATGGCGCTGACGACAGGTGCTGGCGCCGGGT
GTGGCCGCTGGAGATGACGTAGTTTTCGCGCTTAAATTTGAGAAAGGGCGCGAAA
CTAGTCCTTAAGAGTCAGCGCGCAGTATTTGCTGAAGAGAGCCTCCGCGTCTTCCA
GCGTGCGCCGAAGCTGATCTTCGCTTTTGTGATACAGGCAGCTGCGGGTGAGGGAG
CGCAGAGACCTGTTTTTTATTTTCAGCTCTTGTTCTTGGCCCCTGCTTTGTTGAAATA
TAGCATACAGAGTGGGAAAAATCCTATTTCTAAGCTCGCGGGTCGATACGGGTTCG
TTGGGCGCCAGACGCAGCGCTCCTCCTCCTGCTGCTGCCGCCGCTGTGGATTTCTT
GGGCTTTGTCAGAGTCTTGCTATCCGGTCGCCTTTGCTTCTGTGTGACCGCTGCTGT
TGCTGCCGCTGCCGCTGCCGCCGGTGCAGTAGGGGCTGTAGAGATGACGGTAGTAA
TGCAGGATGTTACGGGGGAAGGCCACGCCGTGATGGTAGAGAAGAAAGCGGCGG
GCGAAGGAGATGTTGCCCCCACAGTCTTGCAAGCAAGCAACTATGGCGTTCTTGTG
CCCGCGCCACGAGCGGTAGCCTTGGCGCTGTTGTTGCTCTTGGGCTAACGGCGGCG
GCTGCTTAGACTTACCGGCCCTGGTTCCAGTGGTGTCCCATCTACGGTTGGGTCGG
CGAACAGGCAGTGCCGGCGGCGCCTGAGGAGCGGAGGTTGTAGCGATGCTGGGA
ACGGTTGCCAATTTCTGGGGCGCCGGCGAGGGGAATGCGACCGAGGGTGACGGTG
TTTCGTCTGACACCTCTTCGGCCTCGGAAGCTTCGTCTAGGCTGTCCCAGTCTTCCA
TCATCTCCTCCTCCTCGTCCAAAACCTCCTCTGCCTGACTGTCCCAGTATTCCTCCT
CGTCCGTGGGTGGCGGCGGCGGCAGCTGCAGCTTCTTTTTGGGTGCCATCCTGGGA
 
AGCAAGGGCCCGCGGCTGCTGATAGGGCTGCGGCGGGGGATTGGGTTGAGC
TCCTCGCCGGACTGGGGGTCCAGGTAAACCCCCCGTCCCTTTCGTAGCAGAAACTC
TTGGCGGGCTTTGTTGATGGCTTGCAATTGGCCAAGGATGTGGCCCTGGGTAATGA
CGCAGGCGGTAAGCTCCGCATTTGGCGGGCGGGATTGGTCTTCGTAGAACCTAATC
TCGTGGGCGTGGTAGTCCTCAGGTACAAATTTGCGAAGGTAAGCCGACGTCCACA
GCCCCGGAGTGAGTTTCAACCCCGGAGCCGCGGACTTTTCGTCAGGCGAGGGACC
CTGCAGCTCAAAGGTACCGATAATTTGACTTTCGCTAAGCAGTTGCGAATTGCAGA
CCAGGGAGCGGTGCGGGGTGCATAGGTTGCAGCGACAGTGACACTCCAGTAGGCC
GTCACCGCTCACGTCTTCCATGATGTCGGAGTGGTAGGCAAGGTAGTTGGCTAGCT
GCAGAAGGTAGCAGTGACCCCAAAGCGGCGGAGGGCATTCACGGTACTTAATGGG
CACAAAGTCGCTAGGAAGCGCACAGCAGGTGGCGGGCAGAATTCCTGAACGCTCT
AGGATAAAGTTCCTAAAGTTTTGCAACATGCTTTGACTGGTGAAGTCTGGCAGACC
CTGTTGCAGGGTTTTAAGCAGGCGTTCGGGGAAGATAATGTCCGCCAGGTGCGCGG
CCACGGAGCGCTCGTTGAAGGCCGTCCATAGGTCCTTCAAGTTTTGCTTTAGCAGC
TTCTGCAGCTCCTTTAGGTTGCGCTCCTCCAGGCATTGCTGCCACACGCCCATGGCC
GTTTGCCAGGTGTAGCACAGAAATAAGTAAACGCAGTCGCGGACGTAGTCGCGGC
GCGCCTCGCCCTTGAGCGTGGAATGAAGCACGTTTTGCCCGAGGCGGTTTTCGTGC
AAAATTCCAAGGTAGGAGACCAGGTTGCAGAGCTCCACGTTGGAAATTTTGCAGG
CCTGGCGCACGTAGCCCTGGCGAAAGGTGTAGTGCAACGTTTCCTCTAGCTTGCGC
TGCATCTCCGGGTCAGCAAAGAACCGCTGCATGCACTCAAGCTCCACGGTAACAA
GCACTGCGGCCATCATTAGCTTGCGTCGCTCCTCCAAGTCGGCAGGCTCGCGCGTC
TCAAGCCAGCGCGCCAGCTGCTCATCGCCAACTGCGGGTAGGCCCTCCTCGGTTTG
TTCTTGCAAGTTTGCATCCCTCTCCAGGGGTCGTGCACGGCGCACGATCAGCTCGC
TCATGACTGTGCTCATAACCTTGGGGGGTAGGTTAAGTGCCGGGTAGGCAAAGTGG
GTGACCTCGATGCTGCGTTTCAGCACGGCTAGGCGCGCGTTGTCACCCTCAAGTTC
CACCAGCACTCCACAGTGACTTTCATTTTCGCTGTTTTCTTGTTGCAGAGCGTTTGC
CGCGCGTTTCTCGTCGCGTCCAAGACCCTCAAAGATTTTTGGCACTTCGTCGAGCG
AGGCGATATCAGGTATGACAGCGCCCTGCCGCAAGGCCAGCTGCTTGTCCGCTCGG
CTGCGGTTGGCACGGCAGGATAGGGGTATCTTGCAGTTTTGGAAAAAGATGTGATA
GGTGGCAAGCACCTCTGGCACGGCAAATACGGGGTAGAAGTTGAGGCGCGGGTTG
GGCTCGCATGTGCCGTTTTCTTGGCGTTTGGGGGGTACGCGCGGTGAGAACAGGTG
GCGTTCGTAGGCAAGGCTGACATCCGCTATGGCGAGGGGCACATCGCTGCGCTCTT
GCAACGCGTCGCAGATAATGGCGCACTGGCGCTGCAGATGCTTCAACAGCACGTC
 
GTCTCCCACATCTAGGTAGTCGCCATGCCTTTGGTCCCCCCGCCCGACTTGTTCCTC
GTTTGCCTCTGCGTCGTCCTGGTCTTGCTTTTTATCCTCTGTTGGTACTGAGCGATCC
TCGTCGTCTTCGCTTACAAAACCTGGGTCCTGCTCGATAATCACTTCCTCCTCCTCA
AGCGGGGGTGCCTCGACGGGGAAGGTGGTAGGCGCGTTGGCGGCATCGGTGGAG
GCGGTGGTGGCGAACTCAAAGGGGGCGGTTAGGCTGTCCTCCTTCTCGACTGACT
CCATGATCTTTTTCTGCCTATAGGAGAAGGAAATGGCCAGTCGGGAAGAGGAGCAG
CGCGAAACCACCCCCGAGCGCGGACGCGGTGCGGCGCGACGTCCACCAACCATGG
AGGACGTGTCGTCCCCGTCGCCGTCGCCGCCGCCTCCCCGCGCGCCCCCAAAAAA
GCGGCTGAGGCGGCGTCTCGAGTCCGAGGACGAAGAAGACTCGTCACAAGATGC
GCTGGTGCCGCGCACACCCAGCCCGCGGCCATCGACCTCGACGGCGGATTTGGCC
ATTGCGTCCAAAAAGAAAAAGAAGCGCCCCTCTCCCAAGCCCGAGCGCCCGCCAT
CCCCAGAGGTGATCGTGGACAGCGAGGAAGAAAGAGAAGATGTGGCGCTACAAAT
GGTGGGTTTCAGCAACCCACCGGTGCTAATCAAGCACGGCAAGGGAGGTAAGCGC
ACGGTGCGGCGGCTGAATGAAGACGACCCAGTGGCGCGGGGTATGCGGACGCAAG
AGGAAAAGGAAGAGTCCAGTGAAGCGGAAAGTGAAAGCACGGTGATAAACCCGC
TGAGCCTGCCGATCGTGTCTGCGTGGGAGAAGGGCATGGAGGCTGCGCGCGCGTT
GATGGACAAGTACCACGTGGATAACGATCTAAAGGCAAACTTCAAGCTACTGCCTG
ACCAAGTGGAAGCTCTGGCGGCCGTATGCAAGACCTGGCTAAACGAGGAGCACCG
CGGGTTGCAGCTGACCTTCACCAGCAACAAGACCTTTGTGACGATGATGGGGCGAT
TCCTGCAGGCGTACCTGCAGTCGTTTGCAGAGGTAACCTACAAGCACCACGAGCC
CACGGGCTGCGCGTTGTGGCTGCACCGCTGCGCTGAGATCGAAGGCGAGCTTAAG
TGTCTACACGGGAGCATTATGATAAATAAGGAGCACGTGATTGAAATGGATGTGAC
GAGCGAAAACGGGCAGCGCGCGCTGAAGGAGCAGTCTAGCAAGGCCAAGATCGT
GAAGAACCGGTGGGGCCGAAATGTGGTGCAGATCTCCAACACCGACGCAAGGTGC
TGCGTGCATGACGCGGCCTGTCCGGCCAATCAGTTTTCCGGCAAGTCTTGCGGCAT
GTTCTTCTCTGAAGGCGCAAAGGCTCAGGTGGCTTTTAAGCAGATCAAGGCTTTCA
TGCAGGCGCTGTATCCTAACGCCCAGACCGGGCACGGTCACCTTCTGATGCCACTA
CGGTGCGAGTGCAACTCAAAGCCTGGGCATGCACCCTTTTTGGGAAGGCAGCTAC
CAAAGTTGACTCCGTTCGCCCTGAGCAACGCGGAGGACCTGGACGCGGATCTGAT
CTCCGACAAGAGCGTGCTGGCCAGCGTGCACCACCCGGCGCTGATAGTGTTCCAG
TGCTGCAACCCTGTGTATCGCAACTCGCGCGCGCAGGGCGGAGGCCCCAACTGCG
ACTTCAAGATATCGGCGCCCGACCTGCTAAACGCGTTGGTGATGGTGCGCAGCCTG
TGGAGTGAAAACTTCACCGAGCTGCCGCGGATGGTTGTGCCTGAGTTTAAGTGGA
 
GCACTAAACACCAGTATCGCAACGTGTCCCTGCCAGTGGCGCATAGCGATGCGCGG
CAGAACCCCTTTGATTTTTAAACGGCGCAGACGGCAAGGGTGGGGGGTAAATAATC
ACCCGAGAGTGTACAAATAAAAACATTTGCCTTTATTGAAAGTGTCTCCTAGTACAT
TATTTTTACATGTTTTTCAAGTGACAAAAAGAAGTGGCGCTCCTAATCTGCGCACTG
TGGCTGCGGAAGTAGGGCGAGTGGCGCTCCAGGAAGCTGTAGAGCTGTTCCTGGT
TGCGACGCAGGGTGGGCTGTACCTGGGGACTGTTAAGCATGGAGTTGGGTACCCC
ATAGAGCCCACCGCATCGAGGCTGATCAGCGGGTTTAAACGGGCCCTCTAGACTCG
AGCGGCCGCCTACATGGGGGTAGAGTCATAATCGTGCATCAGGATAGGGCGGTGGT
GCTGCAGCAGCGCGCGAATAAACTGCTGCCGCCGCCGCTCCGTCCTGCAGGAATAC
AACATGGCAGTGGTCTCCTCAGCGATGATTCGCACCGCCCGCAGCATGAGACGCCT
TGTCCTCCGGGCACAGCAGCGCACCCTGATCTCACTTAAATCAGCACAGTAACTGC
AGCACAGCACCACAATATTGTTCAAAATCCCACAGTGCAAGGCGCTGTATCCAAAG
CTCATGGCGGGGACCACAGAACCCACGTGGCCATCATACCACAAGCGCAGGTAGA
TTAAGTGGCGACCCCTCATAAACACGCTGGACATAAACATTACCTCTTTTGGCATGT
TGTAATTCACCACCTCCCGGTACCATATAAACCTCTGATTAAACATGGCGCCATCCA
CCACCATCCTAAACCAGCTGGCCAAAACCTGCCCGCCGGCTATGCACTGCAGGGA
ACCGGGACTGGAACAATGACAGTGGAGAGCCCAGGACTCGTAACCATGGATCATC
ATGCTCGTCATGATATCAATGTTGGCACAACACAGGCACACGTGCATACACTTCCTC
AGGATTACAAGCTCCTCCCGCGTCAGAACCATATCCCAGGGAACAACCCATTCCTG
AATCAGCGTAAATCCCACACTGCAGGGAAGACCTCGCACGTAACTCACGTTGTGCA
TTGTCAAAGTGTTACATTCGGGCAGCAGCGGATGATCCTCCAGTATGGTAGCGCGG
GTCTCTGTCTCAAAAGGAGGTAGGCGATCCCTACTGTACGGAGTGCGCCGAGACA
ACCGAGATCGTGTTGGTCGTAGTGTCATGCCAAATGGAACGCCGGACGTAGTCATA
TTTCCTCGACGATCTCTATCACTGATAGGGAGATCTCTATCACTGATAGGGAGAGCT
CTGCTTATATAGACCTCCCACCGTACACGCCTACCGCCCATTTGCGTCAATGGGGCG
GAGTTGTTACGACATTTTGGAAAGTCCCGTTGATTTTGGTTCCAAAACAAACTCCC
ATTGACGTCAATGGGGTGGAGACTTGGAAATCCCCGTGAGTCAAACCGCTATCCAC
GCCCATTGATGTACTGCCAAAACCGCATCACATGGTGCACTCTCAGTACAATCATCC
GTATTGGGATGCGGCCGCAATATTTGCATGTCGCTATGTGTTCTGGGAAATCACCAT
AAACGTGAAATCCCTATCAGTGATAGAGACTTATAAGTTCCCTATCAGTGATAGAGA
ACCGGTGGGCACTCTTCCGTGGTCTGGTGGATAAATTCGCAAGGGTATCATGGCGG
ACGACCGGGGTTCGAACCCCGGATCCGGCCGTCCGCCGTGATCCATGCGGTTACCG
CCCGCGTGTCGAACCCAGGTGTGCGACGTCAGACAACGGGGGAGCGCTCCTTTTT
 
GAATTCCACTTTGGCCGCGGCTCGAGGACATTGATTATTGACTAGTTATTAATAGTA
ATCAATTACGGGGTCATTAGTTCATAGCCCATATATGGAGTTCCGCGTTACATAACTT
ACGGTAAATGGCCCGCCTGGCTGACCGCCCAACGACCCCCGCCCATTGACGTCAAT
AATGACGTATGTTCCCATAGTAACGCCAATAGGGACTTTCCATTGACGTCAATGGGT
GGAGTATTTACGGTAAACTGCCCACTTGGCAGTACATCAAGTGTATCATATGCCAAG
TACGCCCCCTATTGACGTCAATGACGGTAAATGGCCCGCCTGGCATTATGCCCAGTA
CATGACCTTATGGGACTTTCCTACTTGGCAGTACATCTACGTATTAGTCATCGCTATT
ACCATGGTGATGCGGTTTTGGCAGTACATCAATGGGCGTGGATAGCGGTTTGACTC
ACGGGGATTTCCAAGTCTCCACCCCATTGACGTCAATGGGAGTTTGTTTTGGCACC
AAAATCAACGGGACTTTCCAAAATGTCGTAACAACTCCGCCCCATTGACGCAAATG
GGCGGTAGGCGTGTACGGTGGGAGGTCTATATAAGCAGAGCTCGTTTAGTGAACCG
TCAGATCGCCTGGAGACGCCATCCACGCTGTTTTGACCTCCATAGAAGACACCGGG
ACCGATCCAGCCTCCGCGGCCCCGAATTCACCATGTCTAGATTAGATAAAAGTAAA
GTGATTAACAGCGCATTAGAGCTGCTTAATGAGGTCGGAATCGAAGGTTTAACAAC
CCGTAAACTCGCCCAGAAGCTAGGTGTAGAGCAGCCTACATTGTATTGGCACGTGC
GCAACAAGCAGACTCTTATGAACATGCTTTCAGAGGCAATACTGGCGAAGCATCAC
ACCCGTTCAGCACCGTTACCGACTGAGAGTTGGCAGCAGTTTCTCCAGGAAAATGC
TCTGAGTTTCCGTAAAGCATTACTGGTCCATCGTGATGGAGCCCGATTGCATATAGG
GACCTCTCCTACGCCCCCCCAGTTTGAACAAGCAGAGGCGCAACTACGCTGTCTAT
GCGATGCAGGGTTTTCGGTCGAGGAGGCTCTTTTCATTCTGCAATCTATCAGCCATT
TTACGTTGGGTGCAGTATTAGAGGAGCAAGCAACAAACCAGATAGAAAATAATCAT
GTGATAGACGCTGCACCACCATTATTACAAGAGGCATTTAATATTCAGGCGAGAACC
TCTGCTGAAATGGCCTTCCATTTCGGGCTGAAATCATTAATATTTGGATTTTCTGCAC
AGTTAGATGAAAAAAAGCATACACCCATTGAGGATGGTAATAAACCAAAAAAGAA
GAGAAAGCTAGCAGTGTCAGTGACATTTGAAGATGTGGCTGTGCTCTTTACTCGGG
ACGAGTGGAAGAAGCTGGATCTGTCTCAGAGAAGCCTGTACCGTGAGGTGATGCT
GGAGAATTACAGCAACCTGGCCTCCATGGCAGGATTCCTGTTTACCAAACCAAAGG
TGATCTCCCTGTTGCAGCAAGGAGAGGATCCCTGGTAAATCGATCTGAGAACTTCA
GGGTGAGTTTGGGGACCCTTGATTGTTCTTTCTTTTTCGCTATTGTAAAATTCATGTT
ATATGGAGGGGGCAAAGTTTTCAGGGTGTTGTTTAGAATGGGAAGATGTCCCTTGT
ATCACCATGGACCCTCATGATAATTTTGTTTCTTTCACTTTCTACTCTGTTGACAACC
ATTGTCTCCTCTTATTTTCTTTTCATTTTCTGTAACTTTTTCGTTAAACTTTAGCTTGC
ATTTGTAACGAATTTTTAAATTCACTTTTGTTTATTTGTCAGATTGTAAGTACTTTCTC
 
TAATCACTTTTTTTTCAAGGCAATCAGGGTATATTATATTGTACTTCAGCACAGTTTT
AGAGAACAATTGTTATAATTAAATGATAAGGTAGAATATTTCTGCATATAAATTCTGG
CTGGCGTGGAAATATTCTTATTGGTAGAAACAACTACACCCTGGTCATCATCCTGCC
TTTCTCTTTATGGTTACAATGATATACACTGTTTGAGATGAGGATAAAATACTCTGAG
TCCAAACCGGGCCCCTCTGCTAACCATGTTCATGCCTTCTTCTCTTTCCTACAGCTC
CTGGGCAACGTGCTGGTTGTTGTGCTGTCTCATCATTTTGGCAAAGAATTCACTCCT
CAGGTGCAGGCTGCCTATCAGAAGGTGGTGGCTGGTGTGGCCAATGCCCTGGCTCA
CAAATACCACTGAGATCTTTTTCCCTCTGCCAAAAATTATGGGGACATCATGAAGCC
CCTTGAGCATCTGACTTCTGGCTAATAAAGGAAATTTATTTTCATTGCAATAGTGTGT
TGGAATTTTTTGTGTCTCTCACTCTAGAGCGGCCGCGGATCCAATTCTACCGGGTAG
GGGAGGCGCTTTTCCCAAGGCAGTCTGGAGCATGCGCTTTAGCAGCCCCGCTGGG
CACTTGGCGCTACACAAGTGGCCTCTGGCCTCGCACACATTCCACATCCACCGGTA
GGCGCCAACCGGCTCCGTTCTTTGGTGGCCCCTTCGCGCCACCTTCTACTCCTCCC
CTAGTCAGGAAGTTCCCCCCCGCCCCGCAGCTCGCGTCGTGCAGGACGTGACAAA
TGGAAGTAGCACGTCTCACTAGTCTCGTGCAGATGGACAGCACCGCTGAGCAATG
GAAGCGGGTAGGCCTTTGGGGCAGCGGCCAATAGCAGCTTTGCTCCTTCGCTTTCT
GGGCTCAGAGGCTGGGAAGGGGTGGGTCCGGGGGCGGGCTCAGGGGCGGGCTCA
GGGGCGGGGCGGGCGCCCGAAGGTCCTCCGGAGGCCCGGCATTCTGCACGCTTCA
AAAGCGCACGTCTGCCGCGCTGTTCTCCTCTTCCTCATCTCCGGGCCTTTCGACCTG
CAGTTAACGCCGCCATGACCGAGTACAAGCCCACGGTGCGCCTCGCCACCCGCGA
CGACGTCCCCAGGGCCGTACGCACCCTCGCCGCCGCGTTCGCCGACTACCCCGCCA
CGCGCCACACCGTCGATCCGGACCGCCACATCGAGCGGGTCACCGAGCTGCAAGA
ACTCTTCCTCACGCGCGTCGGGCTCGACATCGGCAAGGTGTGGGTCGCGGACGAC
GGCGCCGCGGTGGCGGTCTGGACCACGCCGGAGAGCGTCGAAGCGGGGGCGGTG
TTCGCCGAGATCGGCCCGCGCATGGCCGAGTTGAGCGGTTCCCGGCTGGCCGCGC
AGCAACAGATGGAAGGCCTCCTGGCGCCGCACCGGCCCAAGGAGCCCGCGTGGTT
CCTGGCCACCGTCGGCGTCTCGCCCGACCACCAGGGCAAGGGTCTGGGCAGCGCC
GTCGTGCTCCCCGGAGTGGAGGCGGCCGAGCGCGCCGGGGTGCCCGCCTTCCTGG
AGACCTCCGCGCCCCGCAACCTCCCCTTCTACGAGCGGCTCGGCTTCACCGTCACC
GCCGACGTCGAGGTGCCCGAAGGACCGCGCACCTGGTGCATGACCCGCAAGCCCG
GTGCCTGACCGCGTCTGGAACAATCAACCTCTGGATTACAAAATTTGTGAAAGATT
GACTGGTATTCTTAACTATGTTGCTCCTTTTACGCTATGTGGATACGCTGCTTTAATG
CCTTTGTATCATGCTATTGCTTCCCGTATGGCTTTCATTTTCTCCTCCTTGTATAAATC
 
CTGGTTGCTGTCTCTTTATGAGGAGTTGTGGCCCGTTGTCAGGCAACGTGGCGTGG
TGTGCACTGTGTTTGCTGACGCAACCCCCACTGGTTGGGGCATTGCCACCACCTGT
CAGCTCCTTTCCGGGACTTTCGCTTTCCCCCTCCCTATTGCCACGGCGGAACTCATC
GCCGCCTGCCTTGCCCGCTGCTGGACAGGGGCTCGGCTGTTGGGCACTGACAATTC
CGTGGTGTTGTCGGGGAAGCTGACGTCCTTTCCATGGCTGCTCGCCTGTGTTGCCA
CCTGGATTCTGCGCGGGACGTCCTTCTGCTACGTCCCTTCGGCCCTCAATCCAGCG
GACCTTCCTTCCCGCGGCCTGCTGCCGGCTCTGCGGCCTCTTCCGCGTCTTCGCCTT
CGCCCTCAGACGAGTCGGATCTCCCTTTGGGCCGCCTCCCCGCAAAATGACCGACC
AAGCGACGCCCAACCTGCCATCACGAGATTTCGATTCCACCGCCGCCTTCTATGAA
AGGTTGGGCTTCGGAATCGTTTTCCGGGACGCCGGCTGGATGATCCTCCAGCGCGG
GGATCTCATGCTGGAGTTCTTCGCCCACCCCAACTTGTTTATTGCAGCTTATAATGG
TTACAAATAAAGCAATAGCATCACAAATTTCACAAATAAAGCATTTTTTTCACTGCA
TTCTAGTTGTGGTTTGTCCAAACTCATCAATGTATCTTATCATGTCTGTAGCTGATCA
ATTGGCGCGCCGAATTCGTTATCTGCAGAATTCGGCTTGAGCGCGCCAGCGCTCGT
TCAAACCTCCCGCTTCAAAATGGAGACCCTGCGTGCTCACTCGGGCCCCCCCCCCC
AGCGAGACCACATGGTGTCGCAAAATGTCGCAAAACACTCACGTGACCTCTAATAC
AGGACCTCCCTAACCCTATGACGTAATTCACGTCACGACTCCACCCCTCCGCCCGC
TGTTTAAACGCCCGGGCTGTAGTTAATGATTAACCCGCCATGCTACTTATCTACGTAG
CCATGGAAACTAGATAAGAAAGAAATACGCAGAGACCAAAGTTCAACTGAAACGA
ATTAAACGGTTTATTGATTAACAAGCAATTAAAGGGGTCGGGTAAGGTATCGGGTTC
CGATAGGTCTGGTGGTTCTGTATTCCCCGGTGCTGTCCGGGGCAAAGTCCACAAAC
TGGGGGTCGTTGTAGTTGTTTGTGTACTGGATCTCTGGGTTCCACCTCTTGGAGTTT
TCCTTCTTGAGCTCCCACTCCATCTCCACGGTGACCTGCCCGGTGCTGTACTGGGTG
ATGAAGCTGCTGACGGGCACGTCCGAGAAGCTGGTGATATTTCCGGGCACAGGCG
TGTTCTTGATGAGCATCATGGGCGGTGGGTGTTTGAGTCCGAATCCGCCCATGGCC
GGAGAGGGGTGAAAGTGCGCCCCCGTCTCTGGGATCTTGGCCCAGATGGGTCCTT
GGAGGTACACGTCCCTCTCCATCCACACGCTGCCGGGCACGATTTCCTGGAGGTTG
TACGTGCCGGTCGCGGGGGCAGTGGTGGAGCTCTGGTTGTTGGTGGCCATCTGCCC
GCCGACGTTGTACGCCACGCGGTTCACCGGCTGCGTCTCGCTCTCGCTGGTGATGA
GCATGTTGCCCTCGAGGTACGTGGCGGTGGTGCCCGGGTTCGCCGGCTGGCTGTTG
AAGATCATAGTGTTCTCCAGGGCATAGGTGTTGCTGCCCTGGAGGTTGTTGGTCATG
CCGTTCGGCTGCGGGGGCACCTGGTAACTCGCGCCCTCGAGCTCCATCCTATTGGT
CGTGGCGAAGGCGCTGACACTGGCGCGGTTGACCCCGGAGCCCAGGTTCCAGCCC
 
TGGGTTCGGCCCATGGGCCCCGGGAACCAGTTTTTGTAGGTGTTGGCGTATCTCCC
GGCCAGGTTCTTGTTGAACTGGACTCCGCCAGTGTTATTTGTGCTCACGAAGCGGT
ACAAGTACTGGTCCACCAGCGGGTTGGCCAGCTTGAACAGGTTCTGACTGGGAGC
GAAGCTGGAGTGGAAGGGCACCTCCTCAAAGTTGTAGGTAAACTCAAAGTTGTTG
CCCGTTCTCAGCATCTTGCTGGGAAAGTACTCTAGGCAGAAGAAGCTGCTCCTCTC
GGTGGGATTTTCTGTGTTGTCGCGGTTCAGCGTCGCGTAACCGTACTGCGGCAGCG
TAAAGACCTGCGGAGGGAAGGCCGGCAGGCATCCCTCGGTCCCGTTGCCGACGAC
GTAGGGCAGCTGGTAGTCGTCGTCCGTAAACACTTGGACGGTGGAGGTGAGGTTG
TTGGCGATGGTGGTGGTGGAGTCCTGCACCGTGACCTCTTTGACTTGAATGTTGAA
GATTTTGACTCTGAGGGACCGGGGTCTGAAGCCCCAGTAGTTGTTGATGAGTCTTT
GCCAGTCTCGGGGGCTCCAGTGGCTGTGGAAGCGGTTAAAGTCAAAGTACCCCCA
GGGGGTGCTGTATCCAAAGTAGGCGTTGGCGTTGCTTCCGTCGACGGAGCCGCTTT
TGATCTCTCGGTACTGGTGGTTGTTGTAGCTGGGCAGCACCCAGGTTCGGGTGGAC
TTGGTGACGACTCTGTCCCCCATCCACGTGGAATCGCAATGCCAATCTCCCGAGGC
ATTGCCCACTCCATCGGCACCTTGGTTATTGTCGCCCAATGGGCCGCCACCTCCCGC
AGACATTGTATCAGCTCCCAAACTTGAGGCTGGTTGGGCTGGGATTTGCAGCTGCT
GGGATCCGCTGGGTCCAGCTTCGGCGTCTGACGAGGTGGAAGGCTTGGAGTCCTC
TTCGGTCCGAGCCTTCTTTCTTTTTGGAAAGTGGTCGTCTATCCGCTTTCCGGTAGG
GGCCGTCTTAGCACCCTCTTCAACCAGGCCAAAAGGTTCGAGAACCCTTTTCTTGG
CCTGAAAGACTGCCTTTCCGAGGTTTCCCCCGAAGGATGTGTCGTCGGCGAGCTTC
TCCTGAAACTCGGCGTCCGCGTGGTTGTACTTGAGGTAGGGGTTGTCTCCCGCCTC
AAGCTGCTCGTTGTACGAGATGTCGTGCTCTCGCGCGACCTCGTCTGCCCTGTTGA
CAGGCTCTCCTCGATCGAGACCGTTTCCGGGTCCGAGATAGTTATAACCAGGCAGC
ACAAGACCACGGGCTTGATCTTGATGCTGCTGATTGGGTTTTGGTTTCGGTGGGCC
CGCTTCAAGGCCCAAAAACTCGCGAAGACCTTCACCAACTTCTTCCAACCAATCTG
GAGGGTGATCAACAAAAGACATACCTGATTTAAATCATTTATTGTTCAAAGATGCAG
TCATCCAAATCCACATTGACCAGATCGCAGGCAGTGCAAGCGTCTGGCACCTTTCC
CATGATATGATGAATGTAGCACAGTTTCTGATACGCCTTTTTGACGACAGAAACGGG
TTGAGATTCTGACACGGGAAAGCACTCTAAACAGTCTTTCTGTCCGTGAGTGAAGC
AGATATTTGAATTCTGATTCATTCTCTCGCATTGTCTGCAGGGAAACAGCATCAGAT
TCATGCCCACGTGACGAGAACATTTGTTTTGGTACCTGTCTGCGTAGTTGATCGAAG
CTTCCGCGTCTGACGTCGATGGCTGCGCAACTGACTCGCGCACCCGTTTGGGCTCA
CTTATATCTGCGTCACTGGGGGCGGGTCTTTTCTTGGCTCCACCCTTTTTGACGTAG
 
AATTCATGCTCCACCTCAACCACGTGATCCTTTGCCCACCGGAAAAAGTCTTTGAC
TTCCTGCTTGGTGACCTTCCCAAAGTCATGATCCAGACGGCGGGTGAGTTCAAATT
TGAACATCCGGTCTTGCAACGGCTGCTGGTGTTCGAAGGTCGTTGAGTTCCCGTCA
ATCACGGCGCACATGTTGGTGTTGGAGGTGACGATCACGGGAGTCGGGTCTATCTG
GGCCGAGGACTTGCATTTCTGGTCCACGCGCACCTTGCTTCCTCCGAGAATGGCTT
TGGCCGACTCCACGACCTTGGCGGTCATCTTCCCCTCCTCCCACCAGATCACCATCT
TGTCGACACAGTCGTTGAAGGGAAAGTTCTCATTGGTCCAGTTTACGCACCCGTAG
AAGGGCACAGTGTGGGCTATGGCCTCCGCGATGTTGGTCTTCCCGGTAGTTGCAGG
CCCAAACAGCCAGATGGTGTTCCTCTTGCCGAACTTTTTCGTGGCCCATCCCAGAA
AGACGGAAGCCGCATATTGGGGATCGTACCCGTTTAGTTCCAAAATTTTATAAATCC
GATTGCTGGAAATGTCCTCCACGGGCTGCTGGCCCACCAGGTAGTCGGGGGCGGTT
TTAGTCAGGCTCATAATCTTTCCCGCATTGTCCAAGGCAGCCTTGATTTGGGACCGC
GAGTTGGAGGCCGCATTGAAGGAGATGTATGAGGCCTGGTCCTCCTGGATCCACTG
CTTCTCCGAGGTAATCCCCTTGTCCACGAGCCACCCGACCAGCTCCATGTACCTGG
CTGAAGTTTTTGATCTGATCACCGGCGCATCAGAATTGGGATTCTGATTCTCTTTGT
TCTGCTCCTGCGTCTGCGACACGTGCGTCAGATGCTGCGCCACCAACCGTTTACGC
TCCGTGAGATTCAAACAGGCGCTTAAATACTGTTCCATATTAGTCCACGCCCACTGG
AGCTCAGGCTGGGTTTTGGGGAGCAAGTAATTGGGGATGTAGCACTCATCCACCAC
CTTGTTCCCGCCTCCGGCGCCATTTCTGGTCTTTGTGACCGCGAACCAGTTTGGCA
AAGTCGGCTCGATCCCGCGGTAAATTCTCTGAATCAGTTTTTCGCGAATCTGACTCA
GGAAACGTCCCAAAACCATGGATTTCACCCCGGTGGTTTCCACGAGCACGTGCATG
TGGAAGTAGCTCTCTCCCTTCTCAAATTGCACAAAGAAAAGAGCCTCCGGGGCCTT
ACTCACACGGCGCCATTCCGTCAGAAAGTCGCGCTGCAGCTTCTCGGCCACGGTC
AGGGGTGCCTGCTCAATCAGATTCAGATCCATGTCAGAATCTGGCGGCAACTCCCA
TTCCTTCTCGGCCACCCAGTTCACAAAGCTGTCAGAAATGCCGGGCAGATGCTCGT
CAAGGTCGCTGGGGACCTTAATCACAATCTCGTAAAACCCCGGCATGGCGGCTGCG
CGTTCAAACCTCCCGCTTCAAAATGGAGACCCTGCGTGCTCACTCGGGCTTAAATA
GAATGGCTAGGATCCGGCCGGCCTGCAGGTGTCCTCACAGGAACGAAGTCCCTAA
AGAAACAGTGGCAGCCAGGTTTAGCCCCGGAATTGACTGGATTCCTTTTTTAGGGC
CCATTGGTATGGCTTTTTCCCCGTATCCCCCCAGGTGTCTGCAGGCTCAAAGAGCAG
CGAGAAGCGTTCAGAGGAAAGCGATCCCGTGCCACCTTCCCCGTGCCCGGGCTGT
CCCCGCACGCTGCCGGCTCGGGGATGCGGGGGGAGCGCCGGACCGGAGCGGAGC
CCCGGGCGGCTCGCTGCTGCCCCCTAGCGGGGGAGGGACGTAATTACATCCCTGGG
 
GGCTTTGGGGGGGGGCTGTCCCTCTAGAGCGGCCGCCACCGCGGTGGAGCTCCAG
CTTTTGTTCCCTTTAGTGAGGGTTAATTAGATCTTAATACGACTCACTATAGGGCGAA
TTGGGTACCGGGCCCCCCCTCGAGGTCGACGGTATCGCCTCCAAGGCCAGCTTCCC
ACAATAAGTTGGGTGAATTTTGGCTCATTCCTCCTTTCTATAGGATTGAGGTCAGAG
CTTTGTGATGGGAATTCTGTGGAATGTGTGTCAGTTAGGGTGTGGAAAGTCCCGCG
ATCGCTAGCAAACGCCAGCAACGCGGCCTTTTTACGGTTCCTGGCCTTTTGCTGGC
CTTTTGCTCACATGTCCTGCAGGCAGCTGCGCGCTCGCTCGCTCACTGAGGCCGCC
CGGGCAAAGCCCGGGCGTCGGGCGACCTTTGGTCGCCCGGCCTCAGTGAGCGAGC
GAGCGCGCAGAGAGGGAGTGGCCAACTCCATCACTAGGGGTTCCTGCGGCCGCAC
GCGTGGAGCTAGTTATTAATAGTAATCAATTACGGGGTCATTAGTTCATAGCCCATAT
ATGGAGTTCCGCGTTACATAACTTACGGTAAATGGCCCGCCTGGCTGACCGCCCAA
CGACCCCCGCCCATTGACGTCAATAATGACGTATGTTCCCATAGTAACGTCAATAGG
GACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAAT
GGCCCGCCTGGCATTATGCCCAGTACATGACCTTATGGGACTTTCCTACTTGGCAGT
ACATCTACGTATTAGTCATCGCTATTACCATGGTGATGCGGTTTTGGCAGTACATCAA
TGGGCGTGGATAGCGGTTTGACTCACGGGGATTTCCAAGTCTCCACCCCATTGACG
TCAATGGGAGTTTGTTTTGCACCAAAATCAACGGGACTTTCCAAAATGTCGTAACA
ACTCCGCCCCATTGACGCAAATGGGCGGTAGGCGTGTACGGTGGGAGGTCTATATA
AGCAGAGCTCGTTTAGTGAACCGTCAGATCGCCTGGAGACGCCATCCACGCTGTTT
TGACCTCCATAGAAGACACCGGGACCGATCCAGCCTCCGCGGATTCGAATCCCGGC
CGGGAACGGTGCATTGGAACGCGGATTCCCCGTGCCAAGAGTGACGTAAGTACCG
CCTATAGAGTCTATAGGCCCACAAAAAATGCTTTCTTCTTTTAATATACTTTTTTGTTT
ATCTTATTTCTAATACTTTCCCTAATCTCTTTCTTTCAGGGCAATAATGATACAATGTA
TCATGCCTCTTTGCACCATTCTAAAGAATAACAGTGATAATTTCTGGGTTAAGGCAA
TAGCAATATTTCTGCATATAAATATTTCTGCATATAAATTGTAACTGATGTAAGAGGTT
TCATATTGCTAATAGCAGCTACAATCCAGCTACCATTCTGCTTTTATTTTATGGTTGG
GATAAGGCTGGATTATTCTGAGTCCAAGCTAGGCCCTTTTGCTAATCATGTTCATACC
TCTTATCTTCCTCCCACAGCTCCTGGGCAACGTGCTGGTCTGTGTGCTGGCCCATCA
CTTTGGCAAAGAATTGGGATTCGAACATCGATTGAATTCTGAATGGTGAGCAAGGG
CGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTACGGCA
AGCTGACCCTGAAGTTCATCTGCACCACCGGCAAGCTGCCCGTGCCCTGGCCCACC
 
CTCGTGACCACCCTGACCTACGGCGTGCAGTGCTTCAGCCGCTACCCCGACCACAT
GAAGCAGCACGACTTCTTCAAGTCCGCCATGCCCGAAGGCTACGTCCAGGAGCGC
ACCATCTTCTTCAAGGACGACGGCAACTACAAGACCCGCGCCGAGGTGAAGTTCG
AGGGCGACACCCTGGTGAACCGCATCGAGCTGAAGGGCATCGACTTCAAGGAGGA
CGGCAACATCCTGGGGCACAAGCTGGAGTACAACTACAACAGCCACAACGTCTAT
ATCATGGCCGACAAGCAGAAGAACGGCATCAAGGTGAACTTCAAGATCCGCCACA
ACATCGAGGACGGCAGCGTGCAGCTCGCCGACCACTACCAGCAGAACACCCCCAT
CGGCGACGGCCCCGTGCTGCTGCCCGACAACCACTACCTGAGCACCCAGTCCGCC
CTGAGCAAAGACCCCAACGAGAAGCGCGATCACATGGTCCTGCTGGAGTTCGTGA
CCGCCGCCGGGATCACTCTCGGCATGGACGAGCTGTACAAGTACTCAGATCTCGAG
CTCAAGTAGGGATCCTCTAGAGTCGACCTGCAGAAGCTTGCCTCGAGCAGCGCTGC
TCGAGAGATCTACGGGTGGCATCCCTGTGACCCCTCCCCAGTGCCTCTCCTGGCCC
TGGAAGTTGCCACTCCAGTGCCCACCAGCCTTGTCCTAATAAAATTAAGTTGCATC
ATTTTGTCTGACTAGGTGTCCTTCTATAATATTATGGGGTGGAGGGGGGTGGTATGG
AGCAAGGGGCAAGTTGGGAAGACAACCTGTAGGGCCTGCGGGGTCTATTGGGAAC
CAAGCTGGAGTGCAGTGGCACAATCTTGGCTCACTGCAATCTCCGCCTCCTGGGTT
CAAGCGATTCTCCTGCCTCAGCCTCCCGAGTTGTTGGGATTCCAGGCATGCATGAC
CAGGCTCAGCTAATTTTTGTTTTTTTGGTAGAGACGGGGTTTCACCATATTGGCCAG
GCTGGTCTCCAACTCCTAATCTCAGGTGATCTACCCACCTTGGCCTCCCAAATTGCT
GGGATTACAGGCGTGAACCACTGCTCCCTTCCCTGTCCTTCTGATTTTGTAGGTAAC
CACGTGCGGACCGAGCGGCCGCAGGAACCCCTAGTGATGGAGTTGGCCACTCCCT
CTCTGCGCGCTCGCTCGCTCACTGAGGCCGGGCGACCAAAGGTCGCCCGACGCCC
GGGCTTTGCCCGGGCGGCCTCAGTGAGCGAGCGAGCGCGCAGCTGCCTGCAGGGG
CGCCTGATGCGGTATTTTCTCCTTACGCATCTGTGCGGTATTTCACACCGCATACGTC
GTAGCTGATCAATTGGCGCGCCGAATTCGTTAACAAGCTTTAATTAACGCGTACGAT
AAGCTTGATATCTATAACAAGAAAATATATATATAATAAGTTATCACGTAAGTAGAAC
ATGAAATAACAATATAATTATCGTATGAGTTAAATCTTAAAAGTCACGTAAAAGATAA
TCATGCGTCATTTTGACTCACGCGGTCGTTATAGTTCAAAATCAGTGACACTTACCG
CATTGACAAGCACGCCTCACGGGAGCTCCAAGCGGCGACTGAGATGTCCTAAATG
CACAGCGACGGATTCGCGCTATTTAGAAAGAGAGAGCAATATTTCAAGAATGCATG
CGTCAATTTTACGCAGACTATCTTTCTAGGGTTAATCTAGCTGCATCAGGATCATATC
GTCGGGTCTTTTTTCCGGCTCAGTCATCGCCCAAGCTGGCGCTATCTGGGCATCGGG
GAGGAAGAAGCCCGTGCCTTTTCCCGCGAGGTTGAAGCGGCATGGAAAGAGTTTG
 
CCGAGGATGACTGCTGCTGCATTGACGTTGAGCGAAAACGCACGTTTACCATGATG
ATTCGGGAAGGTGTGGCCATGCACGCCTTTAACGGTGAACTGTTCGTTCAGGCCAC
CTGGGATACCAGTTCGTCGCGGCTTTTCCGGACACAGTTCCGGATGGTCAGCCCGA
AGCGCATCAGCAACCCGAACAATACCGGCGACAGCCGGAACTGCCGTGCCGGTGT
GCAGATTAATGACAGCGGTGCGGCGCTGGGATATTACGTCAGCGAGGACGGGTATC
CTGGCTGGATGCCGCAGAAATGGACATGGATACCCCGTGAGTTACCCGGCGGGCGC
GCTTGGCGTAATCATGGTCATAGCTGTTTCCTGCTCGTGTCCGATAACTGTAATTATC
GGACATGATTGTGAAATTGTTATCCGCTCACAATTCCACACAACATACGAGCCGGA
AGCATAAAGTGTAAAGCCTGGGGTGCCTAATGAGTGAGCTAACTCACATTAATTGC
GTTGCGCTCACTGCCCGCTTTCCAGTCGGGAAACCTGTCGTGCCAGCTGCATTAAT
GAATCGGCCAACGCGCGGGGAGAGGCGGTTTGCGTATTGGGCGCTCTTCCGCTTCC
TCGCTCACTGACTCGCTGCGCTCGGTCGTTCGGCTGCGGCGAGCGGTATCAGCTCA
CTCAAAGGCGGTAATACGGTTATCCACAGAATCAGGGGATAACGCAGGAAAGAAC
ATGTGAGCAAAAGGCCAGCAAAAGGCCAGGAACCGTAAAAAGGCCGCGTTGCTG
GCGTT
Seq2: 
TCGCGCGTTTCGGTGATGACGGTGAAAACCTCTGACACATGCAGCTCCCG
GAGACTGTCACAGCTTGTCTGTAAGCGGATGCCGGGAGCAGACAAGCCCGTCAGG
GCGCGTCAGCGGGTGTTGGCGGGTGTCGGGGCTGGCTTAACTATGCGGCATCAGA
GCAGATTGTACTGAGAGTGCACCATATGCGGTGTGAAATACCGCACAGATGCGTAA
GGAGAAAATACCGCATCAGGCGCCATTCGCCATTCAGGCTGCGCAACTGTTGGGAA
GGGCGATCGGTGCGGGCCTCTTCGCTATTACGCCAGCTGGCGAAAGGGGGATGTGC
TGCAAGGCGATTAAGTTGGGTAACGCCAGGGTTTTCCCAGTCACGACGTTGTAAAA
CGACGGCCAGTGAATTGACGCGTATTGGGATCCTGCAGGTCGTTACATAACTTACG
GTAAATGGCCCGCCTGGCTGACCGCCCAACGACCCCCGCCCATTGACGTCAATAAT
GACGTATGTTCCCATAGTAACGCCAATAGGGACTTTCCATTGACGTCAATGGGTGGA
GTATTTACGGTAAACTGCCCACTTGGCAGTACATCAAGTGTATCATATGCCAAGTAC
GCCCCCTATTGACGTCAATGACGGTAAATGGCCCGCCTGGCATTATGCCCAGTACAT
GACCTTATGGGACTTTCCTACTTGGCAGTACATCTACGTATTAGTCATCGCTATTACC
ATGGTGATGCGGTTTTGGCAGTACATCAATGGGCGTGGATAGCGGTTTGACTCACG
GGGATTTCCAAGTCTCCACCCCATTGACGTCAATGGGAGTTTGTTTTGGCACCAAA
ATCAACGGGACTTTCCAAAATGTCGTAACAACTCCGCCCCATTGACGCAAATGGGC
GGTAGGCGTGTACGGTGGGAGGTCTATATAAGCAGAGCTCGTTTAGTGAACCGTCA
 
GATCGCCTGGAGACGCCATCCACGCTGTTTTGACCTCCATAGAAGACACCGGGACC
GATCCAGCCTCCGGACTCTAGAGGATCCGGTACTAGAGGAACTGAAAAACCAGAA
AGTTAACTGGTAAGTTTAGTCTTTTTGTCTTTTATTTCAGGTCCCGGATCCGGTGGT
GGTGCAAATCAAAGAACTGCTCCTCAGTGGATGTTGCCTTTACTTCTAGGCCTGTA
CGGAAGTGTTACTTCTGCTCTAAAAGCTGCGGAATTGTACCCGCGGGCCCACCATG
TACCCATACGATGTTCCAGATTACGCTCTTATGGCCATGGAGGCCCGAATTCGGTCG
ACCATGGGTAGTTCTTTAGACGATGAGCATATCCTCTCTGCTCTTCTGCAAAGCGAT
GACGAGCTTGTTGGTGAGGATTCTGACAGTGAAATATCAGATCACGTAAGTGAAGA
TGACGTCCAGAGCGATACAGAAGAAGCGTTTATAGATGAGGTACATGAAGTGCAGC
CAACGTCAAGCGGTAGTGAAATATTAGACGAACAAAATGTTATTGAACAACCAGGT
TCTTCATTGGCTTCTAACAGAATCTTGACCTTGCCACAGAGGACTATTAGAGGTAAG
AATAAACATTGTTGGTCAACTTCAAAGTCCACGAGGCGTAGCCGAGTCTCTGCACT
GAACATTGTCAGATCTCAAAGAGGTCCGACGCGTATGTGCCGCAATATATATGACCC
ACTTTTATGCTTCAAACTATTTTTTACTGATGAGATAATTTCGGAAATTGTAAAATGG
ACAAATGCTGAGATATCATTGAAACGTCGGGAATCTATGACAGGTGCTACATTTCGT
GACACGAATGAAGATGAAATCTATGCTTTCTTTGGTATTCTGGTAATGACAGCAGTG
AGAAAAGATAACCACATGTCCACAGATGACCTCTTTGATCGATCTTTGTCAATGGTG
TACGTCTCTGTAATGAGTCGTGATCGTTTTGATTTTTTGATACGATGTCTTAGAATGG
ATGACAAAAGTATACGGCCCACACTTCGAGAAAACGATGTATTTACTCCTGTTAGA
AAAATATGGGATCTCTTTATCCATCAGTGCATACAAAATTACACTCCAGGGGCTCAT
TTGACCATAGATGAACAGTTACTTGGTTTTAGAGGACGGTGTCCGTTTAGGATGTAT
ATCCCAAACAAGCCAAGTAAGTATGGAATAAAAATCCTCATGATGTGTGACAGTGG
TACGAAGTATATGATAAATGGAATGCCTTATTTGGGAAGAGGAACACAGACCAACG
GAGTACCACTCGGTGAATACTACGTGAAGGAGTTATCAAAGCCTGTGCACGGTAGT
TGTCGTAATATTACGTGTGACAATTGGTTCACCTCAATCCCTTTGGCAAAAAACTTA
CTACAAGAACCGTATAAGTTAACCATTGTGGGAACCGTGCGATCAAACAAACGCGA
GATACCGGAAGTACTGAAAAACAGTCGCTCCAGGCCAGTGGGAACATCGATGTTTT
GTTTTGACGGACCCCTTACTCTCGTCTCATATAAACCGAAGCCAGCTAAGATGGTAT
ACTTATTATCATCTTGTGATGAGGATGCTTCTATCAACGAAAGTACCGGTAAACCGC
AAATGGTTATGTATTATAATCAAACTAAAGGCGGAGTGGACACGCTAGACCAAATGT
GTTCTGTGATGACCTGCAGTAGGAAGACGAATAGGTGGCCTATGGCATTATTGTACG
GAATGATAAACATTGCCTGCATAAATTCTTTTATTATATACAGCCATAATGTCAGTAG
CAAGGGAGAAAAGGTTCAAAGTCGCAAAAAATTTATGAGAAACCTTTACATGAGC
 
CTGACGTCATCGTTTATGCGTAAGCGTTTAGAAGCTCCTACTTTGAAGAGATATTTG
CGCGATAATATCTCTAATATTTTGCCAAATGAAGTGCCTGGTACATCAGATGACAGTA
CTGAAGAGCCAGTAATGAAAAAACGTACTTACTGTACTTACTGCCCCTCTAAAATA
AGGCGAAAGGCAAATGCATCGTGCAAAAAATGCAAAAAAGTTATTTGTCGAGAGC
ATAATATTGATATGTGCCAAAGTTGTTTCTGAGAGATCTCTCGAGGTACCGCGGCCG
CGGGGATCCAGACATGATAAGATACATTGATGAGTTTGGACAAACCACAACTAGAA
TGCAGTGAAAAAAATGCTTTATTTGTGAAATTTGTGATGCTATTGCTTTATTTGTAAC
CATTATAAGCTGCAATAAACAAGTTATCCCAATGGCGCGCCGAGCTTGGCGTAATCA
TGGTCATAGCTGTTTCCTGTGTGAAATTGTTATCCGCTCACAATTCCACACAACATA
CGAGCCGGAAGCATAAAGTGTAAAGCCTGGGGTGCCTAATGAGTGAGCTAACTCA
CATTAATTGCGTTGCGCTCACTGCCCGCTTTCCAGTCGGGAAACCTGTCGTGCCAG
CTGCATTAATGAATCGGCCAACGCGCGGGGAGAGGCGGTTTGCGTATTGGGCGCTG
TTCCGCTTCCTCGCTCACTGACTCGCTGCGCTCGGTCGTTCGGCTGCGGCGAGCGG
TATCAGCTCACTCAAAGGCGGTAATACGGTTATCCACAGAATCAGGGGATAACGCA
GGAAAGAACATGTGAGCAAAAGGCCAGCAAAAGGCCAGGAACCGTAAAAAGGCC
GCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTT
CCCCCTGGAAGCTCCCTCGTGCGCTCTCCTGTTCCGACCCTGCCGCTTACCGGATAC
CTGTCCGCCTTTCTCCCTTCGGGAAGCGTGGCGCTTTCTCATAGCTCACGCTGTAGG
TATCTCAGTTCGGTGTAGGTCGTTCGCTCCAAGCTGGGCTGTGTGCACGAACCCCC
CGTTCAGCCCGACCGCTGCGCCTTATCCGGTAACTATCGTCTTGAGTCCAACCCGGT
AAGACACGACTTATCGCCACTGGCAGCAGCCACTGGTAACAGGATTAGCAGAGCG
AGGTATGTAGGCGGTGCTACAGAGTTCTTGAAGTGGTGGCCTAACTACGGCTACAC
TAGAAGAACAGTATTTGGTATCTGCGCTCTGCTGAAGCCAGTTACCTTCGGAAAAA
GAGTTGGTAGCTCTTGATCCGGCAAACAAACCACCGCTGGTAGCGGTGGTTTTTTT
GTTTGCAAGCAGCAGATTACGCGCAGAAAAAAAGGATCTCAAGAAGATCCTTTGAT
CTTTTCTACGGGGTCTGACGCTCAGTGGAACGAAAACTCACGTTAAGGGATTTTGG
TCATGAGATTATCAAAAAGGATCTTCACCTAGATCCTTTTAAATTAAAAATGAAGTT
TTAAATCAATCTAAAGTATATATGAGTAAACTTGGTCTGACAGTTACCAATGCTTAAT
CAGTGAGGCACCTATCTCAGCGATCTGTCTATTTCGTTCATCCATAGTTGCCTGACT
CCCCGTCGTGTAGATAACTACGATACGGGAGGGCTTACCATCTGGCCCCAGTGCTG
CAATGATACCGCGAGAACCACGCTCACCGGCTCCAGATTTATCAGCAATAAACCAG
CCAGCCGGAAGGGCCGAGCGCAGAAGTGGTCCTGCAACTTTATCCGCCTCCATCC
 
AGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGG
CTTCATTCAGCTCCGGTTCCCAACGATCAAGGCGAGTTACATGATCCCCCATGTTGT
GCAAAAAAGCGGTTAGCTCCTTCGGTCCTCCGATCGTTGTCAGAAGTAAGTTGGCC
GCAGTGTTATCACTCATGGTTATGGCAGCACTGCATAATTCTCTTACTGTCATGCCAT
CCGTAAGATGCTTTTCTGTGACTGGTGAGTACTCAACCAAGTCATTCTGAGAATAGT
GTATGCGGCGACCGAGTTGCTCTTGCCCGGCGTCAATACGGGATAATACCGCGCCA
CATAGCAGAACTTTAAAAGTGCTCATCATTGGAAAACGTTCTTCGGGGCGAAAACT
CTCAAGGATCTTACCGCTGTTGAGATCCAGTTCGATGTAACCCACTCGTGCACCCA
ACTGATCTTCAGCATCTTTTACTTTCACCAGCGTTTCTGGGTGAGCAAAAACAGGA
AGGCAAAATGCCGCAAAAAAGGGAATAAGGGCGACACGGAAATGTTGAATACTCA
TACTCTTCCTTTTTCAATATTATTGAAGCATTTATCAGGGTTATTGTCTCATGAGCGG
ATACATATTTGAATGTATTTAGAAAAATAAACAAATAGGGGTTCCGCGCACATTTCC
CCGAAAAGTGCCACCTGACGTCTAAGAAACCATTATTATCATGACATTAACCTATAA
AAATAGGCGTATCACGAGGCCCTTTTGTC
seq3: GCCCCAACTGCGACTTCA
seq4: CTCCACTTAAACTCAGGCACAAC
seq5: CGCACCATCACCAACGC
seq6: TCCGTGGTCTGGTGGATAAATTC
seq7: GTCTGACGTCGCACACCT
seq8: TCCGCCGTGATCCATGC
seq9: TGGTTTAGGATGGTGGTGGATG
seq10: CGCAGGTAGATTAAGTGGCGA
seq11: GAGGTAATGTTTATGTCCAGCGTG
seq12: TGGGCGTGGACTAATATGGAA
seq13: CCGGCGCATCAGAATTGG
seq14: AGGAGCAGAACAAAGAGAATCAGAA
seq15: TCACCAAGTCCACCCGAAC
seq16: GCGGTTAAAGTCAAAGTACCCC
seq17: GAAGCAACGCCAACGCC
seq18: GGAACCCCTA GTGATGGAGT T
seq19: CGGCCTCAGT GAGCGA
seq20: CACTCCCTCT CTGCGCGCTC G

[Sequence list] International application 22F00823W1JP23030630_4.xml based on International Patent Cooperation Treaty

## Claims

1. A method of producing a target virus, the method comprising:
a virus production step of culturing an animal cell into which an exogenous recombinant nucleic acid encoding a target virus has been introduced, in a culture medium in a state in which a function of BET is suppressed.

2. The method according to claim 1,
wherein transcription by an RNA polymerase III is promoted by suppressing the function of the BET.

3. The method according to claim 1 or 2,
wherein the function of a BET is suppressed by containing the BET inhibitor in the culture medium.

4. The method according to claim 1 or 2,
wherein a dissociation constant: Kd value of a BET inhibitor with respect to any one of BRD2, BRD3, or BRD4 is 150 nmol/L or less.

5. The method according to claim 1 or 2,
wherein a BET inhibitor is at least one selected from the group consisting of (S)-(+)-tert-butyl 2-(4-(4-chlorophenyl)-2,3,9-trimethyl-6H-thieno[3,2-f][1,2,4]triazolo[4,3-a][1,4]diazepin-6-yl)acetate,
2-methoxy-N-(3-methyl-2-oxo-1,2,3,4-tetrahydro-quinazolin-6-yl)-benzenesulfonamide,
N-[6-(3-methanesulfonamide-4-methylphenyl)-3-methyl-[1,2,4]triazolo[4,3-b]pyridazin-8-yl]carbamate,
(4S)-6-(4-chlorophenyl)-N-ethyl-8-methoxy-1-methyl-4H-[1,2,4]triazolo[4,3-a][1,4]benzodiazepine-4-acetamide,
7,3,5-dimethyl-4-isoxazolyl-1,3-dihydro-8-methoxy-1-[1R-1-(2-pyridinyl)ethyl]-2H-imidazo[4,5-c]quinolin-2-one, and
6H-thieno[3,2-f][1,2,4]triazolo[4,3-a][1,4]diazepine-6-acetamide.

6. The method according to claim 1 or 2,
wherein a BET inhibitor is at least one selected from the group consisting of (S)-(+)-tert-butyl 2-(4-(4-chlorophenyl)-2,3,9-trimethyl-6H-thieno[3,2-f][1,2,4]triazolo[4,3-a][1,4]diazepin-6-yl)acetate,
2-methoxy-N-(3-methyl-2-oxo-1,2,3,4-tetrahydro-quinazolin-6-yl)-benzenesulfonamide,
N-[6-(3-methanesulfonamide-4-methylphenyl)-3-methyl-[1,2,4]triazolo[4,3-b]pyridazin-8-yl]carbamate, and
(4S)-6-(4-chlorophenyl)-N-ethyl-8-methoxy-1-methyl-4H-[1,2,4]triazolo[4,3-a][1,4]benzodiazepine-4-acetamide.

7. The method according to claim 3,
wherein a concentration of the BET inhibitor in the culture medium is 0.01 µmol/L to 100 µmol/L.

8. The method according to claim 1 or 2,
wherein the exogenous recombinant nucleic acid is expressed in a constitutive expression system in the animal cell.

9. The method according to claim 1 or 2,
wherein at least one of the exogenous recombinant nucleic acids contains a promoter controlled by an RNA polymerase III.

10. The method according to claim 1 or 2,
wherein the target virus is an adeno-associated virus.

11. The method according to claim 1 or 2,
wherein the exogenous recombinant nucleic acid is a nucleic acid encoding E2A, E4, VA-RNA, Rep, and Cap.

12. The method according to claim 9,
wherein a gene that is transcribed from the promoter controlled by the RNA polymerase III is a VA-RNAI.

13. The method according to claim 1 or 2,
wherein expression of at least one RNA which is a gene transcriptional product of the exogenous recombinant nucleic acid is 10 times or more higher as compared with a case of performing culturing in a state in which the function of the BET is not suppressed.

14. The method according to claim 1 or 2,
wherein the exogenous recombinant nucleic acid includes at least a nucleic acid encoding E2A and VA-RNAI, and an RNA expression ratio between E2A gene and VA-RNAI gene is E2A:VA-RNAI = 1:1 to 1:10.

15. The method according to claim 1 or 2,
wherein the animal cell is an HEK293 cell.

16. The method according to claim 1 or 2,
wherein at least one of the exogenous recombinant nucleic acids contains a nucleic acid sequence that induces expression in a condition-specific manner.

17. The method according to claim 16,
wherein the expression is induced in a condition-specific manner by applying a stimulus that induces the expression in a condition-specific manner.

18. The method according to claim 1 or 2,
wherein a stimulus that induces the expression in a condition-specific manner is that the culture medium contains doxycycline.

19. The method according to claim 1 or 2,
wherein a period during which the culture medium contains a BET inhibitor is 1 hour or more.

20. The method according to claim 1 or 2,
wherein the method includes stopping the suppression of the function of the BET before recovering a produced virus.

21. The method according to claim 20,
wherein the suppression of the function of the BET is stopped by performing culturing in a culture medium from which a BET inhibitor has been reduced or removed.

22. The method according to claim 21,
wherein the culturing is performed in the culture medium from which the BET inhibitor has been reduced or removed for 1 hour or more.

23. The method according to claim 1 or 2,
wherein a culture medium is supplied during the culturing, and the culture medium to be supplied contains doxycycline and a BET inhibitor.

24. The method according to claim 1 or 2,
wherein a cell density at the time of seeding the cells is 1 × 10⁴ cells/mL or more.

25. The method according to claim 1 or 2, further comprising:
a proliferation step of proliferating the cells before the virus production step.

26. The method according to claim 25,
wherein a proliferation step of culturing the cells in a culture medium that does not contain doxycycline and a BET inhibitor until a cell density reaches 1 × 10⁴ cells/mL or more is provided before the virus production step, and
the culture medium in the virus production step contains doxycycline and a BET inhibitor.

27. The method according to claim 1 or 2,
wherein at least a part of the virus production step is perfusion culture.

28. The method according to claim 1 or 2,
wherein a period of the virus production step is 24 hours or more and 30 days or less.

29. The method according to claim 1 or 2,
wherein the method includes recovering the virus.

30. A culture composition comprising:
an animal cell into which an exogenous recombinant nucleic acid encoding a target virus has been introduced and in which a function of BET is suppressed;
a virus; and
culture medium.

31. The culture composition according to claim 30,
wherein the virus is an adeno-associated virus.

32. The culture composition according to claim 30 or 31,
wherein the culture medium contains a BET inhibitor.
